# EUROPEAN PATENT SPECIFICATION

(11) **EP 3 387 988 B1**
(45) Date of publication and mention of the grant of the patent: **28.02.2024**
(21) Application number: 16873148.7
(22) Date of filing: 12.12.2016
(51) Int. Cl.: A61B 5/02, A61B 5/022, A61B 5/16, G16H 50/70, G16H 50/20

(54) **BIOLOGICAL STATE ESTIMATION DEVICE, BIOLOGICAL STATE ESTIMATION METHOD, COMPUTER PROGRAM, AND RECORDING MEDIUM**
VORRICHTUNG ZUR BESTIMMUNG EINES BIOLOGISCHEN STATUS, VERFAHREN ZUR BESTIMMUNG EINES BIOLOGISCHEN STATUS, COMPUTERPROGRAMM UND AUFZEICHNUNGSMEDIUM
DISPOSITIF ET PROCÉDÉ D'ESTIMATION D'ÉTAT BIOLOGIQUE, PROGRAMME INFORMATIQUE ET SUPPORT D'ENREGISTREMENT

(30) Priority: 12.12.2015 JP 2015242756
(43) Date of publication of application: 17.10.2018
(73) Proprietor: Delta Kogyo Co., Ltd., Aki-gun, Hiroshima 735-8501 (JP)
(72) Inventor: FUJITA Etsunori, Aki-gun Hiroshima 735-8501 (JP); OGURA Yumi, Aki-gun Hiroshima 735-8501 (JP); UCHIKAWA Ryuichi, Aki-gun Hiroshima 735-8501 (JP); NAKASHIMA Kanako, Aki-gun Hiroshima 735-8501 (JP)
(74) Representative: Hoffmann Eitle
(86) International application number: PCT/JP2016/086958
(87) International publication number: WO 2017/099256

(56) References cited:
- WO-A1-2015/083846
- WO-A1-2015/083846
- JP-A- 2014 000 178
- US-A1- 2014 343 446

## Description

### Technical Field

The present invention relates to a biological state estimation device which estimates a biological state from a biosignal, and a computer program therefor.

### Background Art

WO 2015/083846 A1 discusses an acoustic and vibration information accumulation mechanism for more accurately detecting bioacoustics signals and biological signals.

In Patent Documents 1 to 5 and so on, the present inventors have proposed arts to detect vibration generated on the body surface of the back in the upper body of a person by a biosignal measurement device and analyze a state of the person. Sound and vibration information arising from cardiac and aortic motions, which is detected from the back of the upper body of a person, is pressure vibration arising from the cardiac and aortic motions, and includes ventricular systolic and diastolic information, information on vascular wall elasticity which serves as an auxiliary pump for the circulation, and information on reflected waves. That is, the sound and vibration information includes information on vibration including a back body surface pulse wave (Aortic Pulse Wave (APW)) of around 1 Hz generated on the back surface due to the cardiac and aortic motions and information on sound conveyed to the back side in accordance with heartbeat ("pseudo heart sound" (in this specification, sound of the heart collected from the back side is referred to as "pseudo heart sound", in contrast to heart sound which is sound of the heart collected from the chest side)). A signal waveform accompanying heart rate variability includes information on neural activities of the sympathetic nervous system and the parasympathetic nervous system, and a signal waveform accompanying aortic oscillation includes information on sympathetic nerve activity.

In Patent Document 1, slide calculation is performed in which a predetermined time width is set in a time-series waveform of back body surface pulse waves (APW) of around 1 Hz extracted from collected biosignals (sound and vibration information), to find a frequency gradient time-series waveform, and according to the tendency of its variation, for example, according to whether its amplitude is on the increase or the decrease, a biological state is estimated. It is also disclosed that, by frequency analysis of the biosignals, power spectra of respective frequencies corresponding to a function regulation signal, a fatigue reception signal, and an activity regulation signal belonging to predetermined frequency bands from a ULF band (ultra low-frequency band) to a VLF band (very low-frequency band) are found, and a state of a person is determined from time-series variations of the respective power spectra. Since the fatigue reception signal indicates a progress degree of fatigue in a normal active state, additionally comparing predominant degrees of the power spectra of the function regulation signal and the activity regulation signal makes it possible to determine the state of a person (a sympathetic nerve predominant state, a parasympathetic nerve predominant state, or the like). It is further disclosed that, with the total value of the power spectra of frequency components corresponding to these three signals being set as 100, time-series distribution ratios of the respective frequency components are found, and the state of a person is determined using time-series variations of the distribution ratios.

As a method of quantifying a biological state, Patent Document 2 proposes an art to represent a biological state as a physical condition map and a sensation map. To create them, the aforesaid back body surface pulse wave (APW) is frequency-analyzed, an analyzed waveform in each target analysis section is displayed on log-log axes, the analyzed waveform is classified into a low-frequency band, an intermediate-frequency band, or a high-frequency band, and according to a gradient of the classified analyzed waveform and the shape of the whole analyzed waveform, the analyzed waveform is scored based on a predetermined criterion, and the results are plotted on coordinate axes. The physical condition map shows a control state of the autonomic nervous system from a viewpoint of the balance between the sympathetic nerves and the parasympathetic nerves, and in the sensation map, a change state of heart rate variability is superimposed on the physical condition map.

Patent Documents 3 to 5 disclose a means for determining a homeostasis function level. For the determination, the means for determining the homeostasis function level uses at least one or more of plus/minus of a differential waveform of a frequency gradient time-series waveform, plus/minus of an integrated waveform obtained by integrating the frequency gradient time-series waveform, absolute values of frequency gradient time-series waveforms obtained by absolute value processing of a frequency gradient time-series waveform found by a zero-cross method and a frequency gradient time-series waveform found by a peak detection method, and so on. By using the combination of these, it is found on which level the homeostasis function is.

### Prior Art Document

### Patent Document

Patent Document 1: Japanese Patent Application Laid-open No. 2011-167362
Patent Document 2: Japanese Patent Application Laid-open No. 2012-239480
Patent Document 3: WO2011/046178
Patent Document 4: Japanese Patent Application Laid-open No. 2014-117425
Patent Document 5: Japanese Patent Application Laid-open No. 2014-223271

### Summary of the Invention

### Problems to Be Solved by the Invention

All of the above-described arts determine the state of a person by analyzing elements that vary due to fluctuations of the bioregulation functions, and are capable of detecting various biological states such as a hypnagogic symptom phenomenon, an imminent sleep phenomenon, a low consciousness traveling state, a homeostasis function level, an initial fatigue state, and feeling determination. However, these arts do not estimate a biological state of a person by finding a pseudo heart sound waveform by processing biosignals which are collected from the trunk and include biological sound and vibration, analyzing the pseudo heart sound waveform, and comparing predetermined waveform components in the pseudo heart sound waveform. Further, they attempt neither detecting a state of blood pressure fluctuation nor detecting a physiological phenomenon accompanied by blood pressure fluctuation, in particular, detecting a desire to void.

The present disclosure was made in consideration of the above, and has an object to provide a biological state estimation device capable of detecting a biological state by analyzing a pseudo heart sound waveform found through the processing of biosignals which are collected from the trunk and include biological sound and vibration, a biological state estimation method therefor, a computer program therefor, and a recording medium, in particular, to provide a biological state estimation device capable of estimating a state of blood pressure fluctuation itself or a physiological phenomenon accompanied by blood pressure fluctuation, in particular, urinary urgency including the presence/absence of a desire to void, a biological state estimation method therefor, a computer program therefor, and a recording medium.

### Means for Solving the Problems

At least the problem of estimating a state of blood pressure fluctuation is solved by the subject-matter of the independent claims. Subject matter discussed throughout the following but not falling under the scope of the claims does not form part of the invention.
In order to solve the aforesaid problem, the biological state estimation device is a biological state estimation device which estimates a biological state by using a biosignal, the biological state estimation device including a biological state estimation means which estimates the biological state by using a pseudo heart sound waveform corresponding to a period of heart sound and comparing predetermined waveform components in the pseudo heart sound waveform, the pseudo heart sound waveform being obtained by processing back biological sound and vibration information which is collected as the biosignal from the back of a person and fluctuates according to a flow rate of blood pumped out from the heart.

The biological state estimation means estimates the biological state by comparing amplitudes of two waveform components included in one cardiac cycle of the pseudo heart sound waveform.

The biological state estimation means plots two amplitudes (i, i+1) of the waveform components in a time-series manner in a coordinate system, with one of the amplitudes taken on the axis of abscissas and the other taken on the axis of ordinates, and estimates the biological state from a variance state of a group of plotted points.

The biological state estimation means estimates the biological state from a gradient of the group of the plotted points.

According to a non-claimed example, , the biological state estimation means estimates the biological state by comparing patterns of amplitude changes in cardiac cycles of the pseudo heart sound waveform.

According to this example, the biological state estimation means classifies the patterns of the amplitude changes in the cardiac cycles of the pseudo heart sound waveform into a positive waveform pattern and a negative waveform pattern with respect to a highest peak where an amplitude is the highest in each of the cardiac cycles, the positive waveform pattern being a pattern in which a lowest bottom where the amplitude is the lowest appears immediately after the highest peak, and the negative waveform pattern being a pattern in which a lowest bottom appears immediately before the highest peak, and estimates the biological state from an appearance ratio of the two waveform patterns in a predetermined time.

The biological state estimation means includes a blood pressure fluctuation estimation means which estimates a state of blood pressure fluctuation as the biological state from the pseudo heart sound waveform.

Preferably, the biological state estimation means includes a physiological phenomenon estimation means which estimates a physiological phenomenon as the biological state from the pseudo heart sound waveform.

Preferably, the physiological phenomenon estimation means is a means which estimates a desire to void.

Further, a computer program is a computer program causing a computer to execute a procedure for estimating a biological state by processing a biosignal, the computer program causing the computer to execute a biological state estimation procedure which estimates the biological state by using a pseudo heart sound waveform corresponding to a period of heart sound and comparing predetermined waveform components in the pseudo heart sound waveform, the pseudo heart sound waveform being obtained by processing back biological sound and vibration information which is collected as the biosignal from the back of a person and fluctuates according to a flow rate of blood pumped out from the heart.

The biological state estimation procedure estimates the biological state by comparing amplitudes of two waveform components included in one cardiac cycle of the pseudo heart sound waveform.

The biological state estimation procedure plots two amplitudes (i, i+1) of the waveform components in a time-series manner in a coordinate system, with one of the amplitudes taken on the axis of abscissas and the other taken on the axis of ordinates, and estimates the biological state from a variance state of a group of plotted points.

The biological state estimation procedure estimates the biological state from a gradient of the group of the plotted points.

According to a non-claimed example, , the biological state estimation procedure estimates the biological state by comparing patterns of amplitude changes in cardiac cycles of the pseudo heart sound waveform.

According to this example, the biological state estimation procedure classifies the patterns of the amplitude changes in the cardiac cycles of the pseudo heart sound waveform into a positive waveform pattern and a negative waveform pattern with respect to a highest peak where an amplitude is the highest in each of the cardiac cycles, the positive waveform pattern being a pattern in which a lowest bottom where the amplitude is the lowest appears immediately after the highest peak, and the negative waveform pattern being a pattern in which the lowest bottom appears immediately before the highest peak, and estimates the biological state from an appearance ratio of the two waveform patterns in a predetermined time.

The biological state estimation procedure executes a blood pressure fluctuation estimation procedure which estimates a state of blood pressure fluctuation as the biological state from the pseudo heart sound waveform.

Preferably, the biological state estimation procedure executes a physiological phenomenon estimation procedure which estimates a physiological phenomenon as the biological state from the pseudo heart sound waveform.

Preferably, the physiological phenomenon estimation procedure executes a procedure which estimates a desire to void.

Further, a non-claimed computer-readable recording medium is provided in which the above-described computer program causing the computer as a biological state estimation device to execute the procedure for estimating the biological state by processing the biosignal is recorded.

Further, a non-claimed biological state estimation method is a biological state estimation method which estimates a biological state by using a biosignal, the method including estimating the biological state by using a pseudo heart sound waveform corresponding to a period of heart sound and comparing predetermined waveform components in the pseudo heart sound waveform, the pseudo heart sound waveform being obtained by processing back biological sound and vibration information which is collected as the biosignal from the back of a person and fluctuates according to a flow rate of blood pumped out from the heart.

Preferably, the biological state estimation method estimates the biological state by comparing amplitudes of two waveform components included in one cardiac cycle of the pseudo heart sound waveform, and preferably, plots two amplitudes (i, i+1) of the waveform components in a time-series manner in a coordinate system, with one of the amplitudes taken on the axis of abscissas and the other taken on the axis of ordinates, and estimates the biological state from a variance state of a group of plotted points. Preferably, the biological state is estimated from a gradient of the group of the plotted points. Preferably, the biological state is estimated by comparing patterns of amplitude changes in cardiac cycles of the pseudo heart sound waveform. Preferably, the patterns of the amplitude changes in the cardiac cycles of the pseudo heart sound waveform are classified into a positive waveform pattern and a negative waveform pattern with respect to a highest peak where an amplitude is the highest in each of the cardiac cycles, the positive waveform pattern being a pattern in which a lowest bottom where the amplitude is the lowest appears immediately after the highest peak, and the negative waveform pattern being a pattern in which the lowest bottom appears immediately before the highest peak, and the biological state is estimated from an appearance ratio of the two waveform patterns in a predetermined time. Preferably, as the biological state, at least one state out of blood pressure fluctuation and a physiological phenomenon including a desire to void is estimated from the pseudo heart sound waveform.

### Effect of the Invention

The present invention uses a time-series waveform of the biosignals (back sound and vibration information) collected from the back of a person and including biological sound and vibration. The back sound and vibration information is pressure vibration arising from cardiac and aortic motions, includes ventricular systolic and diastolic information and information on vascular wall elasticity which serves as an auxiliary pump of the circulation, and is considered as a vibration system including both the damping of viscous damping friction and the damping of solid friction. The back sound and vibration information fluctuates according to a flow rate (stroke volume) of blood pumped out from the heart, and the fluctuation of the flow rate is reflected in the amplitude of the time-series waveform of the back sound and vibration information. That is, the back sound and vibration information reflecting the stroke volume of blood from the heart includes waveform components (pseudo first sound, pseudo second sound) whose period corresponds to a period of a waveform of heart sound collected from the chest side by a phonocardiograph, and by analyzing the waveform having these waveform components (pseudo heart sound waveform), it is possible to detect the biological state of a person. This is particularly suitable for detecting a biological state associated with the blood pressure fluctuation corresponding to the fluctuation of the stroke volume of blood from the heart.

Further, being suitable for estimating the state of the blood pressure fluctuation, this is particularly suitable for estimating a state of a physiological phenomenon accompanied by blood pressure fluctuation. For example, during urine storage, a blood pressure usually tends to rise, and by detecting the state of the blood pressure fluctuation, it is possible to estimate a desire to void.

### Brief Description of Drawings

[FIGs. 1] FIG. 1(a) is an exploded view illustrating an example of a biosignal measurement device which is used in one embodiment of the present invention to measure back sound and vibration information, and FIG. 1(b) is a sectional view of an essential part thereof.
[FIG. 2] FIG. 2 is a diagram schematically illustrating the configuration of a biological state estimation device according to an embodiment of the present invention.
[FIG. 3] is a flowchart of a back sound and vibration information processing procedure which is a computer program functioning as a back sound and vibration information processing means.
[FIGs. 4] FIGs. 4(a) to (f) are charts illustrating time-series waveforms obtained by the back sound and vibration information processing means, a pseudo heart sound waveform calculation means, and a low-frequency time-series waveform calculation means.
[FIG. 5] FIG. 5 is a chart illustrating physical characteristics of subjects of an experimental example 1.
[FIGs. 6] FIGs. 6(a) to (1) are charts illustrating experimental results of a subject C in the experimental example 1.
[FIGs. 7] FIGs. 7(a) to (1) are charts illustrating experimental results of a subject A in the experimental example 1.
[FIGs. 8] FIGs. 8(a) to (1) are charts illustrating experimental results of a subject F in the experimental example 1.
[FIGs. 9] FIGs. 9 are correlation charts of RRI and PPWg-D obtained from pseudo heart sound waveforms, of the subjects in the experimental example 1, (a) being the correlation chart of the subject A, (b) being the correlation chart of a subject B, (c) being the correlation chart of the subject C, (d) being the correlation chart of a subject D, (e) being the correlation chart of a subject E, and (f) being the correlation chart of the subject F.
[FIGs. 10] FIGs. 10 are correlation charts of RRI and PPWg-DRRI calculated using five-minute average values, of the subjects in the experimental example 1, (a) being the correlation chart of the subject A, (b) being the correlation chart of the subject B, (c) being the correlation chart of the subject C, (d) being the correlation chart of the subject D, (e) being the correlation chart of the subject E, and (f) being the correlation chart of the subject F.
[FIGs. 11] FIGs. 11 are correlation charts of PCG-PPWg-D and RRI, of the subjects in the experimental example 1, (a) being the correlation chart of the subject A, (b) being the correlation chart of the subject B, (c) being the correlation chart of the subject C, (d) being the correlation chart of the subject D (indicated as "No data"), (e) being the correlation chart of the subject E (indicated as "No data"), and (f) being the correlation chart of the subject F.
[FIGs. 12] FIGs. 12 are correlation charts of five-second average values of PCG-PPWg-D and RRI, of the subjects in the experimental example 1, (a) being the correlation chart of the subject A, (b) being the correlation chart of the subject B, (c) being the correlation chart of the subject C, (d) being the correlation chart of the subject D (indicated as "No data"), (e) being the correlation chart of the subject E (indicated as "No data"), and (f) being the correlation chart of the subject F.
[FIGs. 13] FIGs. 13 are correlation charts of PPG-2nd and RRI, of the subjects in the experimental example 1, (a) being the correlation chart of the subject A, (b) being the correlation chart of the subject B, (c) being the correlation chart of the subject C, (d) being the correlation chart of the subject D, (e) being the correlation chart of the subject E, and (f) being the correlation chart of the subject F.
[FIGs. 14] FIGs. 14 are correlation charts of five-second average values of PPG-2nd and RRI, of the subjects in the experimental example 1, (a) being the correlation chart of the subject A, (b) being the correlation chart of the subject B, (c) being the correlation chart of the subject C, (d) being the correlation chart of the subject D, (e) being the correlation chart of the subject E, and (f) being the correlation chart of the subject F.
[FIGs. 15] FIGs. 15 are charts illustrating relations between frequencies of PCG-PPWg-D and PPG-2nd which are normalized by the maximum amplitude value with respect to PPWg-D normalized by the maximum amplitude value, and a transfer function, (a) being the chart of the subject A, (b) being the chart of the subject B, (c) being the chart of the subject C, (d) being the chart of the subject D, (e) being the chart of the subject E, and (f) being the chart of the subject F.
[FIG. 16] FIG. 16 is a chart illustrating an example of a time-series waveform of PPWg-D.
[FIG. 17] FIG. 17 is a chart illustrating an example of a variance state of points obtained by plotting adjacent two amplitudes (i, i+1) in a time-series manner in a coordinate system, with one of the amplitudes taken on the axis of abscissas and the other taken on the axis of ordinates.
[FIGs. 18] FIG. 18(a) is a chart illustrating a variance state of points obtained by the same plotting as in FIG. 17, regarding PCG-PPWg-D, and FIG. 18(b) is a chart illustrating a variance state of points obtained by the same plotting as in FIG. 17, regarding PPG-2nd.
[FIGs. 19] FIGs. 19 are charts illustrating relations of a gradient angle of a group of plotted points of amplification (2) in PPWg-D with a blood pressure, (a) being a chart illustrating its relation with the highest pressure, and (b) illustrating its relation with the lowest pressure.
[FIGs. 20] FIGs. 20 are charts illustrating relations of the result of PCG-PPWg-D in FIG. 18(a) with the blood pressure, (a) being a chart illustrating its relation with the highest pressure, and (b) illustrating its relation with the lowest pressure.
[FIGs. 21] FIGs. 21 are charts illustrating relations of the result of PPG-2nd in FIG. 18(b) with the blood pressure, (a) being a chart illustrating its relation with the highest pressure, and (b) illustrating its relation with the lowest pressure.
[FIG. 22] is a diagram schematically illustrating the configuration of a biological state estimation device according to another non-claimed embodiment of the present invention.
[FIG. 23] FIG. 23 is an explanatory chart of an estimation method by a physiological phenomenon estimation means of the biological state estimation device according to the aforesaid other non-claimed embodiment.
[FIGs. 24] FIGs. 24(a), (b) are explanatory charts of a method of estimating a desire to void based on distribution density in a plot.
[FIGs. 25] FIGs. 25(a) to (c) are charts illustrating analysis results of a subject A in an experimental example 2.
[FIGs. 26] FIGs. 26(a) to (e) are estimation results of a desire to void found by the physiological phenomenon estimation means of the aforesaid other non-claimed embodiment.
[FIGs. 27] FIG. 27(a) is a chart illustrating a relation between a desire to void and the highest blood pressure, and FIG. 27(b) is a chart illustrating a relation between the desire to void and the lowest blood pressure.
[FIG. 28] FIG. 28 is an explanatory chart of a method of estimating a desire to void by using an appearance ratio of a negative waveform pattern.
[FIGs. 29] FIGs. 29 are charts illustrating experimental results of the subject A, FIG. 29(a) illustrating subjective evaluations of a desire to void and sleepiness, FIG. 29(b) illustrating the highest blood pressure and the lowest blood pressure, FIG. 29(c) illustrating HF and LF/HF, and FIG. 29(d) illustrating an appearance ratio of a negative waveform pattern.
[FIG. 30] FIG. 30 is an explanatory chart of an example of a determination criterion for determining the presence/absence of a desire to void in the appearance ratio of the negative waveform pattern.
[FIGs. 31] FIGs. 31 are charts illustrating experimental results of a subject B, FIG. 31(a) illustrating subjective evaluations of a desire to void and sleepiness, FIG. 31(b) illustrating the highest blood pressure and the lowest blood pressure, FIG.31(c) illustrating HF and LF/HF, and FIG. 31(d) illustrating an appearance ratio of a negative waveform pattern.
[FIGs. 32] FIGs. 32 are explanatory charts of a method of quickly determining a desire to void from the appearance ratio of the negative waveform pattern.
[FIGs. 33] FIGs. 33 are charts illustrating an example where a desire to void is determined by the method in FIGs. 32.
[FIGs. 34] FIGs. 34(a) to (d) are explanatory charts of another method of estimating a biological state from the appearance ratio of the negative waveform pattern.
[FIGs. 35] FIGs. 35(a) to (f) are charts illustrating an example where a biological state including a desire to void of a subject is determined by the method in FIGs. 34.
[FIGs. 36] FIGs. 36(a) to (f) are charts illustrating another example where the biological state including the desire to void of the same subject as that in FIGs. 35 is determined by the method in FIGs. 34.
[FIGs. 37] FIGs. 37(a) to (f) are charts illustrating an example where a biological state including a desire to void of a different subject is determined by the method in FIGs. 34.
[FIGs. 38] FIGs. 38(a) to (e) are charts illustrating an example where a biological state including a desire to void of still another subject is determined by the method in FIGs. 34.
[FIGs. 39] FIGs. 39(a) to (f) are charts illustrating a case where a biological state including a desire to void of yet another subject having strong sleepiness is determined by the method in FIGs. 34.

### Modes for Carrying out the Invention

The present invention will be hereinafter described in more detail based on embodiments of the present invention illustrated in the drawings. A biosignal collected in the present invention is back sound and vibration information. As described above, the back sound and vibration information is sound and vibration information arising from cardiac and aortic motions, which is detected from the back of the upper body of a person, and includes ventricular systolic and diastolic information, information on vascular wall elasticity which serves as an auxiliary pump of blood circulation, information on elasticity by a blood pressure, and information on reflected waves. Therefore, by processing a time-series waveform of the back sound and vibration information, it is possible to create a pseudo heart sound waveform approximating to a heart sound waveform measured by a phonocardiograph, and by analyzing the pseudo heart sound waveform, it is possible to detect a volume stroke and a state of vascular resistance, that is, a state of blood pressure fluctuation.

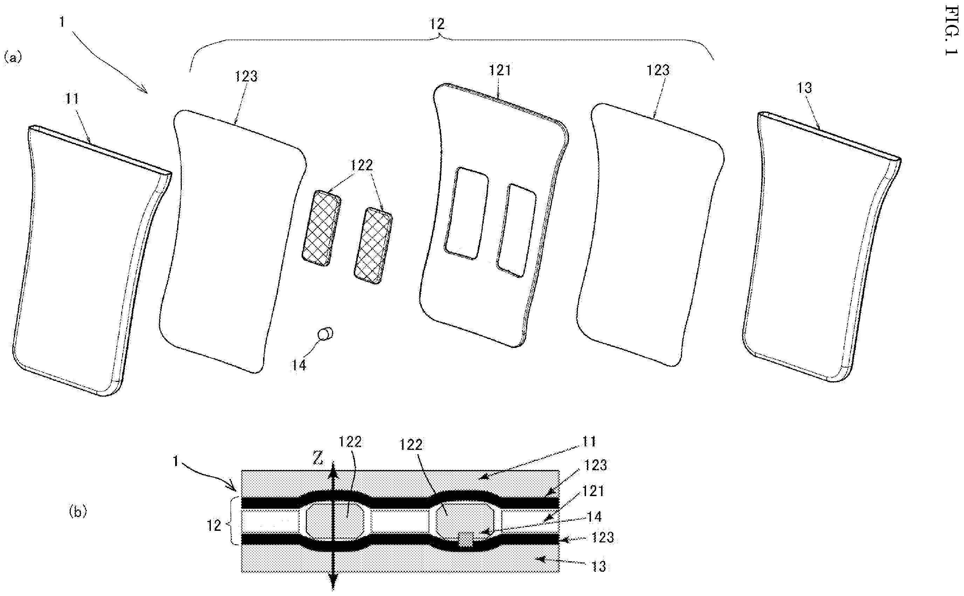

As a biosignal measurement device for collecting the back sound and vibration information, it is preferable to use a biosignal measurement device 1 used in a doze driving warning device (Sleep Buster (registered trademark) manufactured by Delta Tooling Co., Ltd.). FIGs. 1 illustrate the schematic structure of the biosignal measurement device 1. The biosignal measurement device 1 can be assembled in a chair for measurement, a bed, a driver seat of a vehicle, or the like when used, and is capable of collecting biosignals without restraining hands or fingers.

The biosignal measurement device 1 will be briefly described. As illustrated in FIGs. 1(a), (b), the biosignal measurement device 1 has a three-layer structure in which a first layer 11, a second layer 12, and a third layer 13 are stacked in the order mentioned from the top, and when it is used, the first layer 11 formed of a three-dimensional knitted fabric or the like is located on a human body side being a biosignal detection target. Therefore, a biosignal from the back of the trunk of the human body, in particular, the back sound and vibration information including biosound generated in accordance with the vibration of the ventricles, the atrium, and great vessels (direct sound from the trunk or a bioacoustic signal) is propagated first to the first layer 11 being a biosignal input system. The second layer 12 functions as a resonance layer which emphasizes the back sound and vibration information propagated from the first layer 11, by means of a resonance phenomenon or a beat phenomenon, and includes a casing 121 formed of a bead foam or the like, three-dimensional knitted fabrics 122 functioning as natural oscillators, and a film 123 generating membrane vibration. In the second layer 12, a microphone sensor 14 is disposed to detect the back sound and vibration information. The third layer 13 is stacked opposite to the first layer 11 with the second layer 12 therebetween and reduces external sound and vibration input.

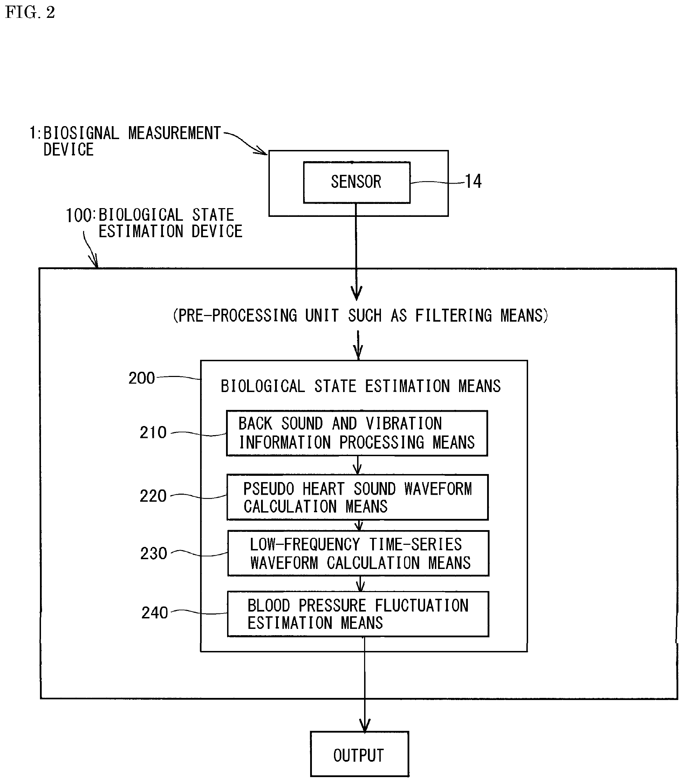

Next, the configuration of a biological state estimation device 100 of this embodiment will be described based on FIG. 2. The biological state estimation device 100 includes a biological state estimation means 200. The biological state estimation means 200 includes a back sound and vibration information processing means 210, a pseudo heart sound waveform calculation means 220, a low-frequency time-series waveform calculation means 230, and a blood pressure fluctuation estimation means 240. The biological state estimation device 100 is constituted by a computer (including a microcomputer or the like), and in its storage, a computer program causing the computer to execute a back sound and vibration information processing procedure, a pseudo heart sound waveform calculation procedure, a low-frequency time-series waveform calculation procedure, and a blood pressure fluctuation estimation procedure which respectively function as the back sound and vibration information processing means 210, the pseudo heart sound waveform calculation means 220, the low-frequency time-series waveform calculation means 230, and the blood pressure fluctuation estimation means 240 to execute a biological state estimation procedure is set. In the biological state estimation means 200, the back sound and vibration information processing means 210, the pseudo heart sound waveform calculation means 220, the low-frequency time-series waveform calculation means 230, and the blood pressure fluctuation estimation means 240 can also be constituted as a back sound and vibration information processing circuit, a pseudo heart sound waveform calculation circuit, a low-frequency time-series waveform calculation circuit, and a blood pressure fluctuation estimation circuit which are electronic circuits caused to operate in predetermined procedures by the aforesaid computer program. It goes without saying that, in the following description, structures named with "means" other than the biological state estimation means 200, the back sound and vibration information processing means 210, the pseudo heart sound waveform calculation means 220, the low-frequency time-series waveform calculation means 230, and the blood pressure fluctuation estimation means 240 can also be constituted as electronic circuit components.

The computer program may be stored in a computer-readable recording medium. By using the recording medium, it is possible to, for example, install the aforesaid program in the aforesaid computer. Here, the recording medium in which the aforesaid program is stored may be a non-transitory recording medium. The non-transitory recording medium is not limited, and examples thereof include recording mediums such as a flexible disk, a hard disk, CD-ROM, MO (magneto-optical disk), DVD-ROM, and a memory card. It is also possible to install the aforesaid program by transmitting it to the aforesaid computer via communication lines.

The back sound and vibration information processing means 210 is a means which applies predetermined processing to the back sound and vibration information obtained from the sensor 14 of the biosignal measurement device 1 (hereinafter, this back sound and vibration information will be referred to as an "original waveform" but the original waveform mentioned here also includes a waveform obtained after a component not used in an analysis, such as body motion, is pre-processed by filtering or the like)), to process the back sound and vibration information into a pseudo heart sound waveform.

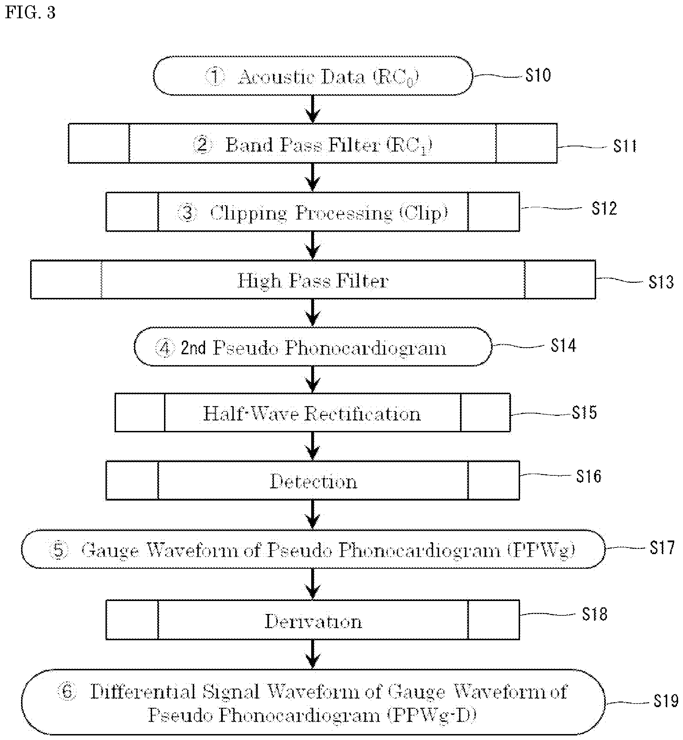

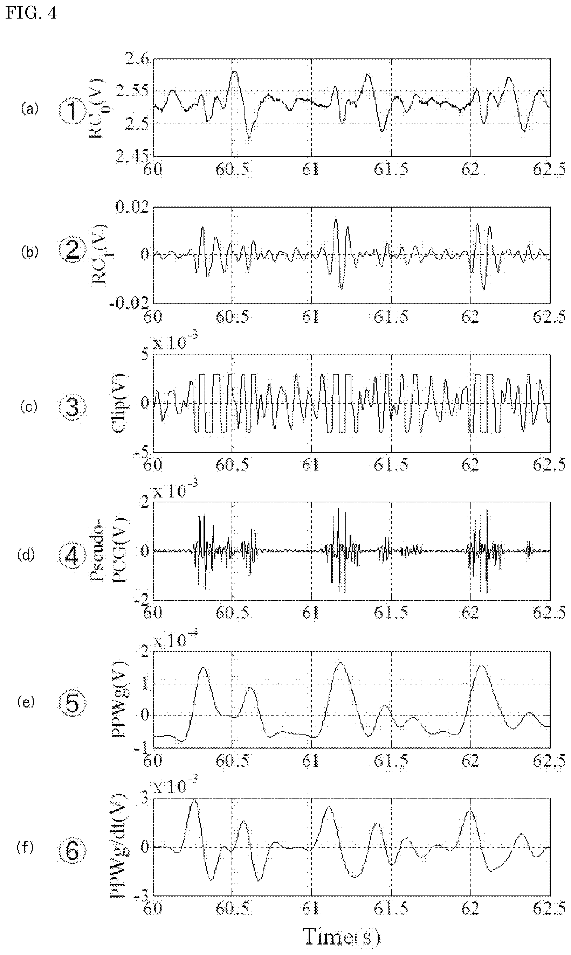

Specifically, the back sound and vibration information processing procedure which is a computer program functioning as the back sound and vibration information processing means 210 is executed by the steps shown in the flowchart in FIG. 3. First, the original waveform RC0 (waveform in FIG. 4 (a)) of the back sound and vibration information is obtained from the sensor 14 (S10). Next, the original waveform RC0 is filtered by a band pass filter with a center frequency of around 20 Hz, for example, by a band pass filter of 10 to 30 Hz, whereby a waveform RC1 (waveform in FIG. 4(b)) is obtained (S11). In the waveform RC1 resulting from the filtering, waveform components whose amplitudes are relatively large appear every about one-second period as illustrated in FIG. 4(b). A standard range of heart rate is about 1 to 1.5 Hz, the period of the large-amplitude waveform components of the waveform RC1 correspond to a cardiac cycle, and the waveform components include pseudo first and second sounds of the amplitude. Therefore, in the this embodiment, this waveform RC1 is a first pseudo heart sound waveform.

The pseudo heart sound waveform calculation means 220 gives a distortion to the waveform RC1 (first pseudo heart sound waveform) obtained by the back sound and vibration information processing means 210, by applying clipping processing in order to extract periods of the pseudo first and second sounds corresponding to first and second heart sounds in a heart sound waveform, thereby finding a time-series waveform (waveform in FIG. 4(c)) having an odd multiple frequency (S12). Note that a threshold of the amplitude at the time of the clipping processing is set at such a position that a time width corresponding to that between the first and second heart sounds can be secured. Next, by the exclusion of signals except those at clipped parts, the pseudo first and second sounds are emphasized, and in order to make the time-series waveform more approximate to the heart sound waveform, a high pass filter is applied (S13), and a second pseudo heart sound waveform (waveform in FIG. 4(d)) in which the pseudo first and second sounds are made prominent are obtained (S14).

The low-frequency time-series waveform calculation means 230 is a means which, in order for the periods of the pseudo first and second sounds in the second pseudo heart sound waveform (waveform in FIG. 4(d)) found by the pseudo heart sound waveform calculation means 220 to be prominent, converts the second pseudo heart sound waveform into a low-frequency time-series waveform with a predetermined frequency or less (what is called a third pseudo heart sound waveform) (waveforms in FIGs. 4(e), (f)).

Specifically, as illustrated in the flowchart in FIG. 3, the low-frequency time-series waveform calculation procedure which is a computer program functioning as the low-frequency time-series waveform calculation means 230 applies half-wave rectification (S15) and detection (S16) to the second pseudo heart sound waveform to obtain a gauge waveform of pseudo heart sound (Gauge Waveform of Pseudo Phonocardiogram, hereinafter "PPWg", the waveform in FIG. 4(e)) (S17). Note that, in order to detect a state of blood pressure fluctuation of a person, a detection range is set from a dominant frequency of around 1 Hz up to a frequency five times the dominant frequency. This will be also described in an experimental example 1 to be described later. Next, by first-order derivation of PPWg (S18), a first differential signal waveform (hereinafter, referred to as "PPWg-D") of PPWg is obtained (S19).

The blood pressure fluctuation estimation means 240 is a means which estimates the state of the blood pressure fluctuation by analyzing amplitude fluctuation of the above-described pseudo heart sound waveform (the RC1 waveform (waveform in FIG. 4(b)) which is the first pseudo heart sound waveform, or the second pseudo heart sound waveform (waveform in FIG. 4(d)) as claimed, or its processed waveform (waveform in FIG. 4(e) or (f))). As described above, the back sound and vibration information is the biosignal including the biological sound and vibration in the body, and the intensity thereof is reflected in the amplitude of the pseudo heart sound waveform and is influenced by a stroke volume and vascular resistance. Accordingly, by analyzing the amplitude fluctuation of the pseudo heart sound waveform, it is possible to estimate the state of the blood pressure fluctuation which is the fluctuation of the stroke volume and the vascular resistance.

Further, as described above, the back sound and vibration information is pressure vibration arising from the cardiac and aortic motions and includes the ventricular systolic and diastolic information and the information on the vascular wall elasticity which serves as the auxiliary pump of the circulation. Accordingly, the back sound and vibration information can be considered as a vibration system including both the damping of viscous damping friction and the damping of solid friction, and a graphical solution which calculates a damping ratio of a free damped vibration waveform can be applied to this. Specifically, regarding a second derivative, it is known that as the blood pressure becomes higher, its reflected wave increases and its late systolic re-descent wave component (d wave) increases, and thus there is a correlation between d/a (a wave: protosystole positive wave) of the second derivative and a systolic blood pressure (SBP), but the present invention uses, as an alternative index to d/a of the second derivative, a logarithmic amplification factor representing an amplification characteristic of the pseudo heart sound waveform. Then, a method which expresses the logarithmic amplification factor by using only an apparent damping ratio found from self-excited vibration of a single-degree-of-freedom system and calculates a damping ratio of the free damped vibration waveform is applied.

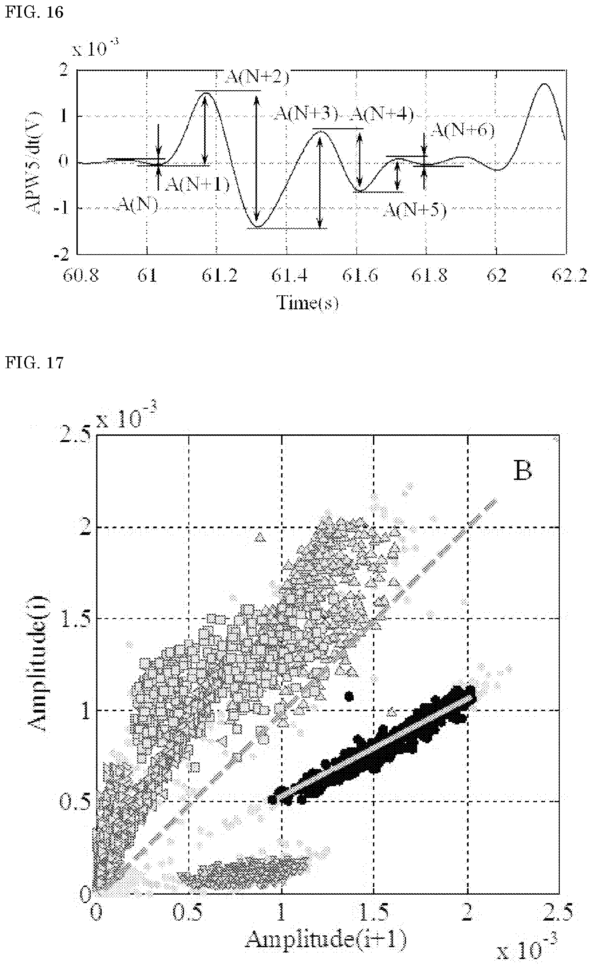

More specifically, the blood pressure fluctuation estimation means 240 plots two adjacent amplitudes (i, i+1) in a period from a starting point of a waveform component of the pseudo first sound up to an end point of a waveform component of the pseudo second sound, which period corresponds to one cardiac cycle, in any one of the aforesaid pseudo heart sound waveforms, in a time-series manner in a coordinate system with one of the amplitudes taken on the axis of abscissas and the other taken on the axis of ordinates, and estimates the state of the blood pressure fluctuation from a variance state of a group of plotted points (refer to FIG. 17).

As described above, the blood pressure fluctuation estimation means 240 according to this embodiment uses, as predetermined waveform components of the pseudo heart sound waveform, the two adjacent amplitudes (i, i+1) in the period from the starting point of the waveform component of the pseudo first sound up to the end point of the waveform component of the pseudo second sound, preferably uses two adjacent amplitudes in an amplification phase of the pseudo first sound as will be described later, to estimate the blood pressure fluctuation. That is, after the extraction of the pseudo heart sound waveform, arithmetic processing necessary for the estimation by the computer is only an analysis of the specific waveform components. Then, by using a gradient angle of an approximate line, it is possible to estimate a relation of thus found variance state of the group of the points indicating an amplitude ratio with respect to the blood pressure as will be described later, and therefore, as compared with a case where change patterns of time-series waveforms are compared, this method leads to a reduction in a load to the computer and an improvement in arithmetic processing speed at the time of the determination, because, if correlation data of the blood pressure and the gradient angle of the approximate line is stored in a storage in advance, it is only necessary to compare the correlation data with the gradient angle of the approximate line of a determination target.

### - Experimental Example 1

### (Experiment Method)

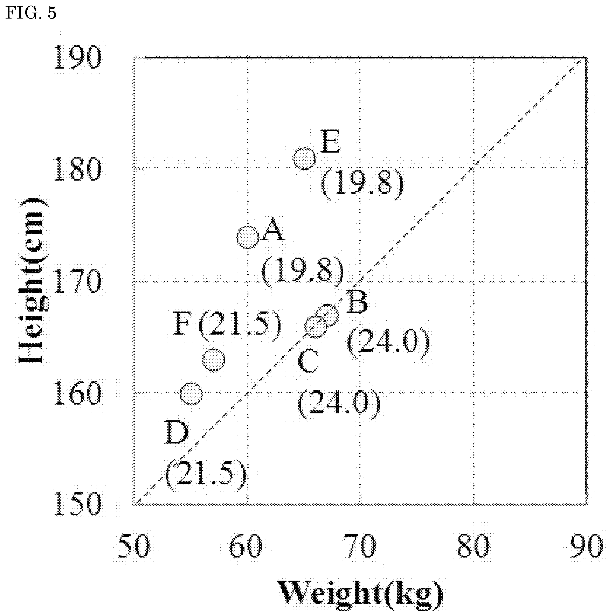

Subjects were each seated in an automobile seat for experiment on whose seat back part the biosignal measurement device 1 was attached as a biosignal measurement device, and back sound and vibration information was collected by the biosignal measurement device 1 while the subjects were in a resting state and a sitting posture. Data of the back sound and vibration information was analyzed by the biological state estimation device 100 which is a computer. At the same time, an electrocardiogram (hereinafter, referred to as "ECC", a measuring instrument: Bedside Monitor BSM-2300 series Life Scope I manufactured by Nihon Kohden Corporation), a phonocardiogram (hereinafter, referred to as "PCG", a measuring instrument: Heart sound/pulse wave amplifiers AS101D and TA701T manufactured by Nihon Kohden Corporation), and a finger plethysmogram (hereinafter, referred to as "PPG", a measuring instrument: Finger Clip Probe SR-5C manufactured by AMCO Inc.) were measured and compared. The measurement by the phonocardiograph was conducted from the front of the chest of each person. The subjects were six healthy male volunteers in their twenties (25.0±2.9 years old) who consented in writing after giving informed consent, and their physical characteristics such as physique were as shown in FIG. 5. The subjects all had an obesity index (BMI value) of 18.5 or more and less than 25 and had standard physique.

The measurement duration was fifteen minutes, and periodic and continuous measurement was conducted with a 1000 Hz sampling frequency by an A/D converter (Power Lab 8/30 manufactured by Nihon Kohden Corporation). Data measured during a five-minute period after the start of the measurement were excluded from measurement targets, and data measured during a period when the subjects were presumed to have been accustomed to a measurement environment, that is, data during a ten-minute period after five minutes passed from the start of the measurement, were used as measurement targets. Further, analysis targets were data during a period in which it was considered that body motion or the like was small and stable data could be measured, that is, data during a 480-second period from sixty seconds up to 540 seconds after five minutes passed from the start of the measurement (that is, a period from six minutes up to fourteen minutes after the start of the measurement). A threshold of the clipping processing executed at Step S12 in FIG. 3 was set to 20% of the maximum value of the amplitude of the RC1 waveform which is the first pseudo heart sound waveform obtained by the processing using the band pass filter at S11. Further, parts where the body motion occurred in behavior records of the subjects during the experiment were excluded from the analysis targets.

Further, after the measurement was conducted for fifteen minutes, a systolic blood pressure (SBP) and a diastolic blood pressure (DBP) of the upper arm were measured using an upper arm blood pressure monitor for home use (OMRON HEM-7051).

### (Experimental Results)

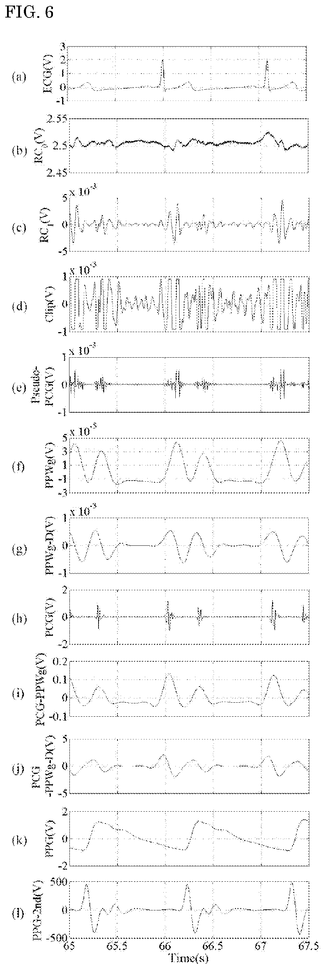

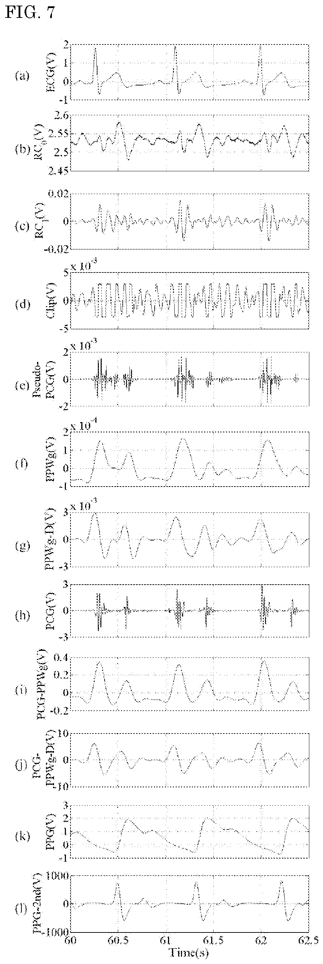

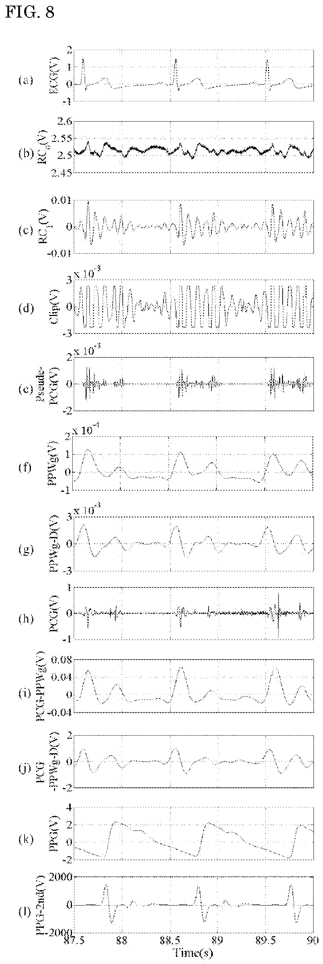

FIGs. 6 to FIGs. 8 illustrate cases of the subject C, the subject A, and the subject F as experimental results. The subject C in FIGs. 6 had a heart rate of 56 beats/minute, SBP of 114 mmgHg, and DBP of 68 mmHg, the subject A in FIGs. 7 had a heart rate of 68 beats/minute, which is the highest among those of all the subjects, SBP of 121 mmgHg, and DBP of 73 mmHg. The subject F in FIGs. 8 had a heart rate of 63 beats/minute, SBP of 111 mmgHg, and DBP of 67 mmHg and thus is a case where both SBP and DBP are low.

In FIGs. 6 to FIGs. 8, (a) illustrates ECG, and (b) is the back sound and vibration information RC0 collected by the biosignal measurement device 1 from the chest back surface which is the back of each person (S10 in FIG. 3). (c) illustrates the RC1 waveform which is the first pseudo heart sound waveform found after the band pass filter of 10 to 30 Hz is applied to RC0 (S11 in FIG. 3), (d) illustrates the waveform resulting from the clipping processing of RC1 (S12 in FIG. 3), (e) illustrates the second pseudo heart sound waveform (including the pseudo first sound and the pseudo second sound) found by further applying the high pass filter whose cutoff frequency is 40 Hz (S13, S14 in FIG. 3). (f) illustrates PPWg found by applying the half-wave rectification to the second pseudo heart sound waveform in (e) (S15 in FIG. 3) and further applying a band pass filter of the dominant frequency (0.93 Hz) to its five multiple frequency (4.65 Hz) (S16, S17 in FIG. 3), and (g) illustrates PPWg-D found through the derivation of PPWg in (f) (S18, S19 in FIG. 3). (h) illustrates a time-series waveform of PCG, (i) illustrates a time-series waveform (hereinafter, "PCG-PPWg") found by applying the half-wave rectification to PCG and further applying a band pass filter of the dominant frequency (0.93 Hz) to its five multiple frequency (4.65 Hz), and (j) illustrates its differential waveform (hereinafter, "PCG-PPWg-D"). (k) illustrates a time-series waveform of PPG, and (1) illustrates a second derivative waveform (hereinafter, "PPG-2nd") of (k).

### (Discussion)

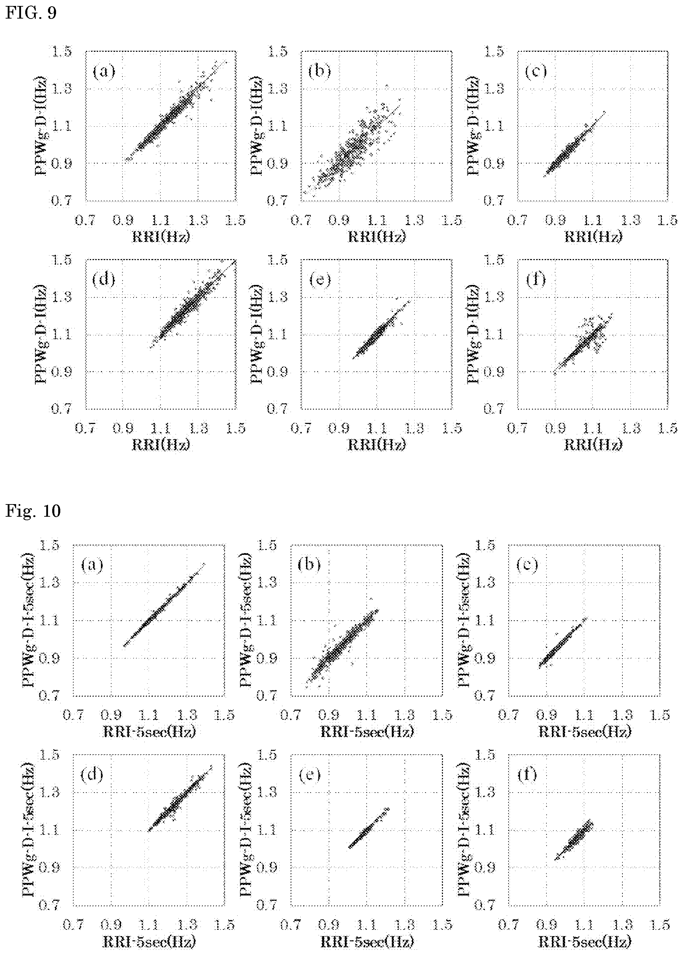

Table 1 shows correlations in cardiac cycle between RRI and PPWg-D obtained from the pseudo heart sound waveforms, of the six subjects A to F. Coefficients of correlation of all the subjects showed a significant correlation, with p < 0.05. FIGs. 9 illustrate correlation charts, with RRI taken on the horizontal axis and PPWg-D taken on the vertical axis.

**[Table 1]**

| | (PPWg-D) | | | | |
|---|---|---|---|---|---|
| Subject | | Corefficient of Correlation | p-value | gradient | intercept |
| Sub. A | | 0.962 | 0.000 | 1.028 | -0.031 |
| Sub. B | | 0.872 | 0.000 | 0.955 | 0.044 |
| Sub. C | | 0.975 | 0.000 | 1.022 | -0.021 |
| Sub. D | | 0.904 | 0.000 | 0.974 | 0.031 |
| Sub. E | | 0.969 | 0.000 | 1.031 | -0.034 |
| Sub. F | | 0.968 | 0.000 | 0.989 | 0.014 |
| Mean ± SD | | 0.942±0.043 | 0.000 | 1.000 ±0.032 | 0.001±0.034 |

Table 2 shows correlations between RRI and PPWg-D obtained from the pseudo heart sound waveforms, which are calculated using mean values of five seconds. Coefficients of correlation of all the subjects showed a significant correlation, with p < 0.05. FIGs. 10 illustrate correlation charts, with RRI taken on the horizontal axis and PPWg-D taken on the vertical axis.

**[Table 2]**

| | (PPWg-D, mean value of 5 seconds) | | | | |
|---|---|---|---|---|---|
| Subject | | Corefficient of Correlation | p-value | gradient | intercept |
| Sub. A | | 0.997 | 0.000 | 1.003 | -0.004 |
| Sub. B | | 0.965 | 0.000 | 0.998 | 0.003 |
| Sub. C | | 0.985 | 0.000 | 1.028 | -0.026 |
| Sub. D | | 0.914 | 0.000 | 0.995 | 0.010 |
| Sub. F | | 0.991 | 0.000 | 1.005 | -0.005 |
| Sub. F | | 0.970 | 0.000 | 0.991 | 0.012 |
| Mean ± SD | | 0.970±0.030 | 0.000 | 1.003±0.013 | -0.001±0.014 |

The cardiac cycle calculated using the mean values of five seconds exhibited the coefficient of correlation and gradient of 0.9 or more in all the subjects. From the above results, it can be said that using the time-series waveforms found from the mean values of five seconds makes it possible to obtain the correlation more significant for biological analysis when heart rate variability is to be detected.

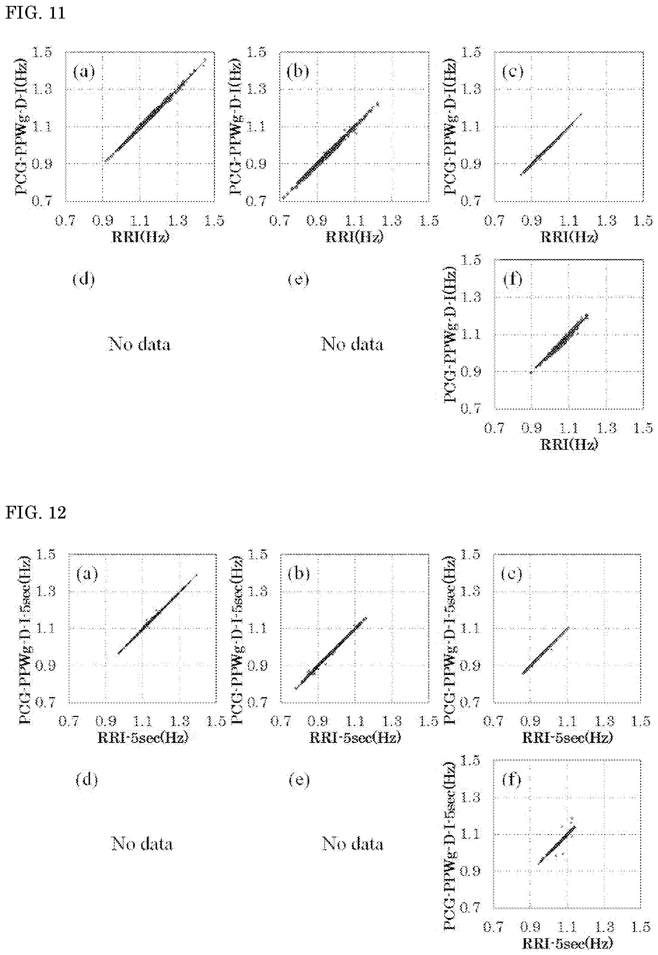

Table 3 shows correlations between PCG-PPWg-D and RRI. FIGs. 11 illustrate correlation charts, with RRI taken on the horizontal axis and PCG-PPWg-D taken on the vertical axis.

**[Table 3]**

| | (PCG-PPWg-D) | | | | |
|---|---|---|---|---|---|
| Subject | | Corefficient of Correlation | p-value | gradient | intercept |
| Sub. A | | 0.998 | 0.000 | 1.012 | -0.014 |
| Sub. Σ3 | | 0.996 | 0.000 | 1.010 | -0.010 |
| Sub. C | | 0.999 | 0.000 | 1.005 | -0.005 |
| Sub. D | | | | - | |
| Sub. E | | | | - | |
| Sub. F | | 0.989 | 0.000 | 1.020 | -0.021 |
| Mean±SD | | 0.995±0.004 | 0.000 | 1.012±0.006 | -0.012±0.007 |

Table 4 shows correlations between five-second mean values of PCG-PPWg-D and RRI. FIGs. 12 illustrate correlation charts, with RRI taken on the horizontal axis and PCG-PPWg-D taken on the vertical axis.

**[Table 4]**

| | (PCG-PPWg-D, mean value of 5 seconds) | | | | |
|---|---|---|---|---|---|
| Subject | | Corefficient of Correlation | p-value | gradient | intercept |
| Sub. A | | 0.999 | 0.000 | 1.003 | -0.003 |
| Sub. B | | 0.999 | 0.000 | 1.002 | -0.002 |
| Sub. C | | 0.999 | 0.000 | 1.002 | -0.002 |
| Sub. D | | | | - | |
| Sub. E | | | | - | |
| Sub. F | | 0.980 | 0.000 | 1.014 | -0.015 |
| Mean ± SD | | 0.994±0.009 | 0.000 | 1.005±0.006 | -0.006±0.006 |

In FIGs. 11 and FIGs. 12, in all the subjects, the coefficient of correlation was 0.9 or more and p < 0.05, and thus a high correlation was exhibited.

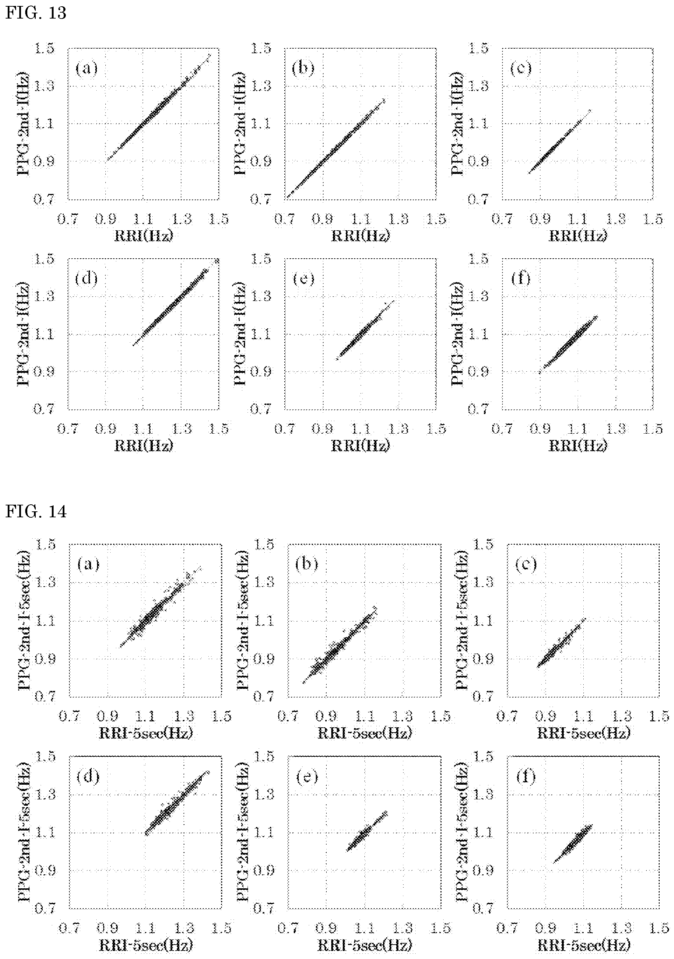

Table 5 shows correlations between PPG-2nd and RRI. FIGs. 13 illustrate correlation charts, with RRI on the horizontal axis and PPG-2nd taken on the vertical axis.

**[Table 5]**

| (Second derivative of PPG : PPG-2nd) | | | | |
|---|---|---|---|---|
| Subject | Corefficient of Correlation | p-value | gradient | intercept |
| Sub. A | 0.997 | 0.900 | 1.012 | -0.013 |
| Sub. B | 0.999 | 0.000 | 1.004 | -0.003 |
| Sub. C | 0.999 | 0.000 | 1.008 | -0.007 |
| Sub. D | 0.997 | 0.000 | 1.014 | -0.017 |
| Sub. E | 0.989 | 0.000 | 1.027 | -0.030 |
| Sub. F | 0.986 | 0.000 | 0.985 | 0.016 |
| Mean ± SD | 0.994±0.005 | 0.000 | 1.008±0.014 | -0.009±0.015 |

In all the subjects, the coefficient of correlation was 0.9 or more and p < 0.05, and thus a high correlation was exhibited.

Table 6 shows correlations between five-second mean values of PPG-2nd and RRI. FIGs. 14 illustrate correlation charts, with RRI taken on the horizontal axis and PPG-2nd taken on the vertical axis.

**[Table 6]**

| | (PPG-2nd, mean value of 5 seconds) | | | | |
|---|---|---|---|---|---|
| Subject | | Corefficient of Correlation | p-value | gradient | intercept |
| Sub. A | | 0.987 | 0.000 | 0.987 | 0.015 |
| Sub. 13 | | 0.987 | 0.000 | 0.983 | 0.016 |
| Sub. C | | 0.982 | 0.000 | 0.979 | 0.020 |
| Sub. I) | | 0.987 | 0.000 | 0.990 | 0.012 |
| Sub. E | | 0.973 | 0.000 | 0.967 | 0.035 |
| Sub. F | | 0.972 | 0.000 | 0.998 | 0.002 |
| Mean±SD | | 0.981 ±0.007 | 0.000 | 0.984±0.011 | 0.017±0.011 |

In all the subjects, the coefficient of correlation was 0.9 or more and p < 0.05, and thus a high correlation was exhibited. However, in PPG-2nd, the coefficient of correlation was 0.994 (Table 5 and FIGs. 13) when values measured every beat were used, and was 0.981 when the mean value of five-second was used (Table 6 and FIGs. 14), and thus the coefficient of correlation was lower when the mean value of five seconds was used. Therefore, in a case of mechanical processing of data in which a delay occurs due to a pulse wave velocity, not using the mean value of five seconds sometimes results in improved accuracy.

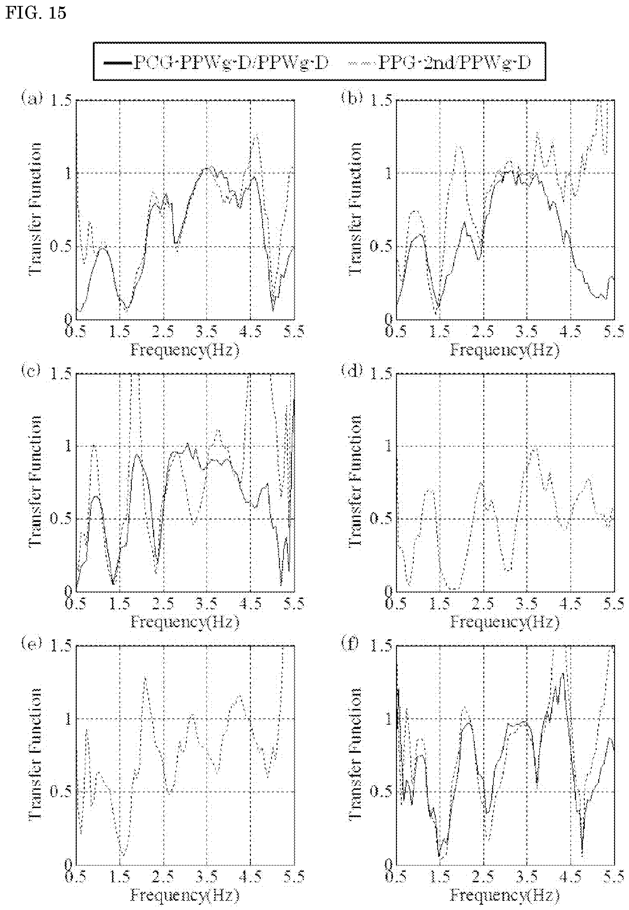

FIGs. 15 illustrate results which are rendered dimensionless by dividing frequency bands of PCG-PPWg-D and PPG-2nd which are normalized by the maximum amplitude value, by frequency bands of PPWg-D normalized by the maximum amplitude value. As the value on the vertical axis is closer to 1, their frequency components are more equal, from which it is thought that the frequency band three to four times the dominant frequency is important, and an appropriate filtering range for obtaining PPWg is 1 to 5 times the dominant frequency.

FIG. 16 illustrates an example (data of the subject C) of the time-series waveform of PPWg-D found by the low-frequency time-series waveform calculation means 230 based on the second pseudo heart sound waveform found by the pseudo heart sound waveform calculation means 220, and FIG. 17 illustrates an example where the blood pressure fluctuation estimation means 240 applies the method of calculating the damping ratio of the free damped vibration waveform to the amplitude fluctuation of the time-series waveform in FIG. 16.

In FIG. 17, A(N) and A(N+1) defined as amplification (1), A(N+1) and A(N+2) defined as amplification (2), A(N+2) and A(N+3) defined as damping (1), A(N+3) and A(N+4) defined as damping (2), and A(N+4) and A(N+5) defined as damping (3) were found every cardiac cycle (from the starting point of the waveform component of the pseudo first sound and the end point of the waveform component of the pseudo second sound) and were plotted. In FIG. 17, the points plotted lower than the broken line B represents the amplification of the amplitude and the points plotted higher than the broken line B represent the damping of the amplitude.

Here, the blood pressure fluctuation estimation means 240 focused on places corresponding to the pseudo first sound when evaluating the variance state of the group of the plotted points. It is known that the first heart sound measured by a phonocardiograph, which corresponds to the pseudo first sound, highly correlates with the blood pressure, and in particular, the amplification phase corresponds to self-excited vibration, and therefore, it is thought that a variance state of a group of plotted points in the amplification phase presents the correlation with the blood pressure. Therefore, the variance state of the group of the plotted points of amplification (2) having the highest peak (in the waveform components in one cardiac cycle, a place where the amplitude had the maximum value on the positive side of the reference line) and exhibiting a noticeable amplifying tendency is focused on, and the biological state is estimated. As the variance state of the group of the plotted points, the blood pressure fluctuation estimation means 240 draws an approximate line by the least square method in the group of the plotted points of amplification (2), calculates its gradient angle with respect to the X-axis, and the correlation with the blood pressure is estimated.

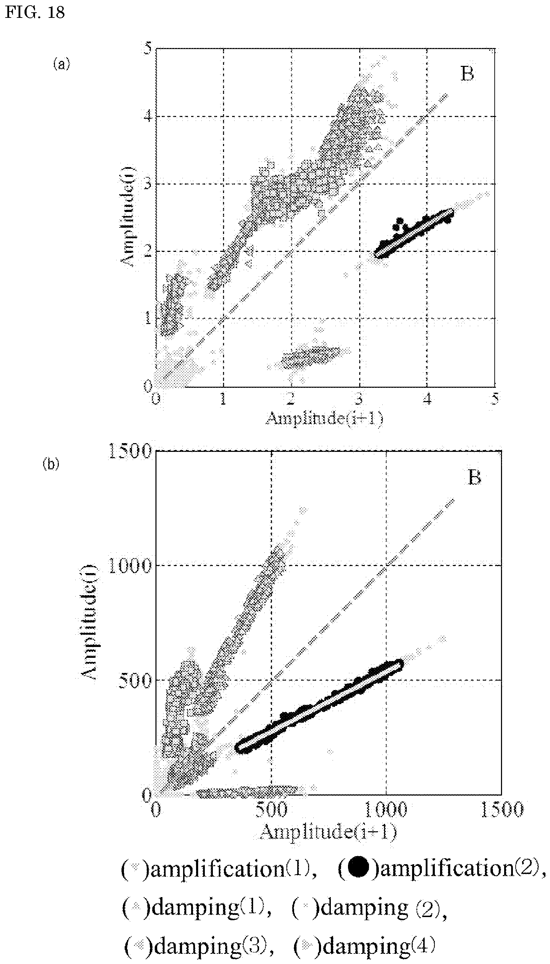

FIG. 18(a) is a chart illustrating a variance state of points plotted by the same processing as in FIG. 17, regarding PCG-PPWg-D, and FIG. 18(b) is a chart illustrating a variance state of points plotted by the same processing as in FIG. 17, regarding PPG-2nd.

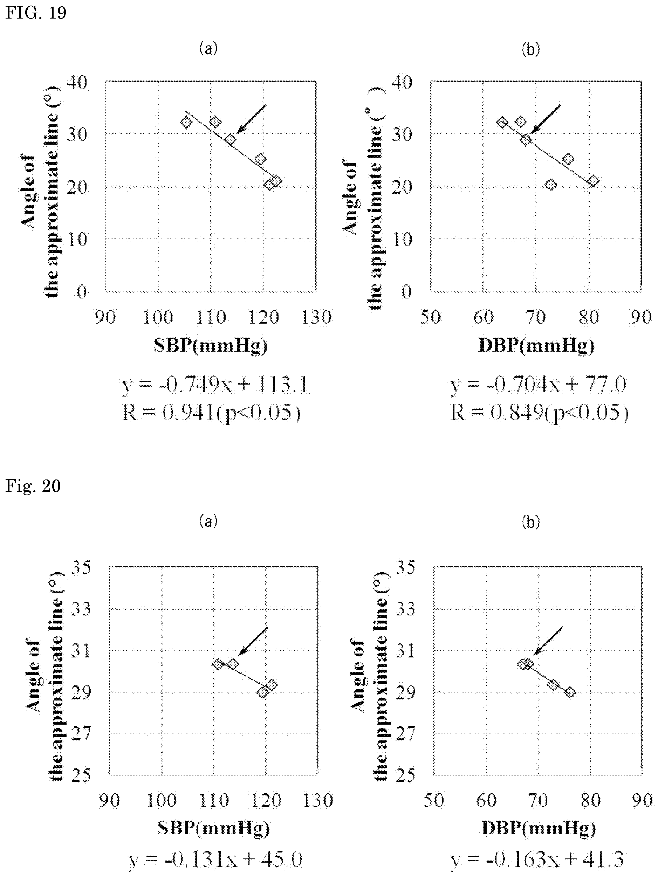

FIGs. 19 are charts illustrating how the gradient angle of the group of the plotted points of amplification (2) in PPWg-D found from the back body surface pulse wave of this embodiment is related with the blood pressure, for all the six subjects. (a) illustrates its relation with the highest pressure and (b) illustrates its relation with the lowest blood pressure. Note that the arrows in the drawings indicate data (gradient angle) of the subject C taken up in FIG. 17. The coefficient of correlation with SBP is 0.941 and the coefficient of correlation with DBP is 0.849, and a high correlation was observed in both of these.

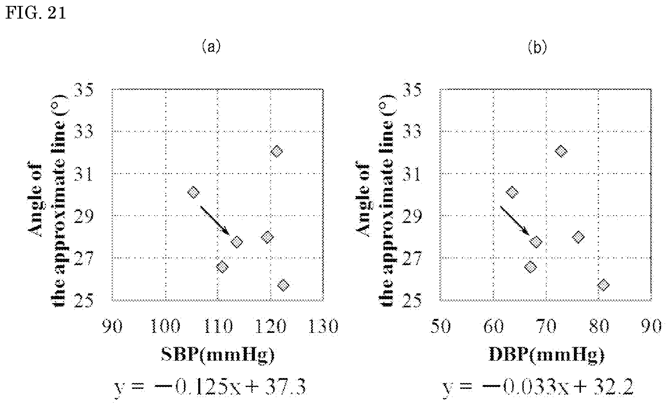

FIGs. 20 are charts illustrating correlations of the result of PCG-PPWg-D with the blood pressure, (a) illustrating its relation with the highest blood pressure and (b) illustrating its relation with the lowest pressure. It is correlated with the blood pressure as in FIGs. 19 found from the back sound and vibration information used in this embodiment. On the other hand, FIGs. 21 are charts illustrating correlations of the result of PPG-2nd with the blood pressure, (a) illustrating its relation with the highest blood pressure and (b) illustrating its relation with the lowest pressure, but it did not have a very high correlation.

Therefore, the use of the method of this embodiment makes it possible to detect the blood pressure fluctuation with accuracy equivalent to that when the phonocardiograph is used, only by measuring the back sound and vibration information, that is, only by seating the subject in a seat where the biosignal measurement device 1 is attached.

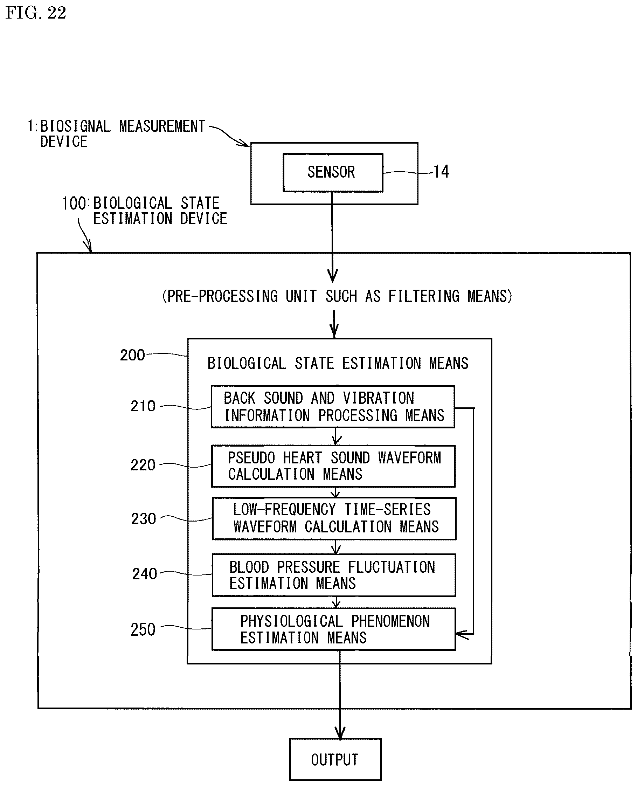

FIG. 22 to FIGs. 27 are explanatory charts of another non-claimed embodiment. As illustrated in FIG. 22, a biological state estimation device 100 of this embodiment further has a physiological phenomenon estimation means 250 as the biological state estimation means 200.

The physiological phenomenon estimation means 250 of this embodiment estimates a physiological phenomenon highly correlating with blood pressure fluctuation. Specifically, it estimates a desire to void typically accompanied by a blood pressure rise phenomenon as urinary storage progresses.

First, the back sound and vibration information processing means 210 applies a band pass filter whose center frequency is near 20 Hz, for example, a band pass filter of 10 to 30 Hz to the measured back sound and vibration information (RC0), to obtain the waveform RC1 which is the first pseudo heart sound waveform (refer to Steps S10 and S11 in FIG. 3).

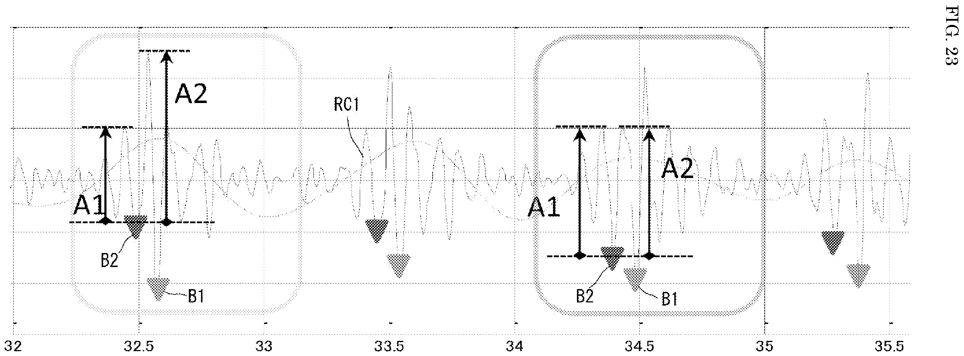

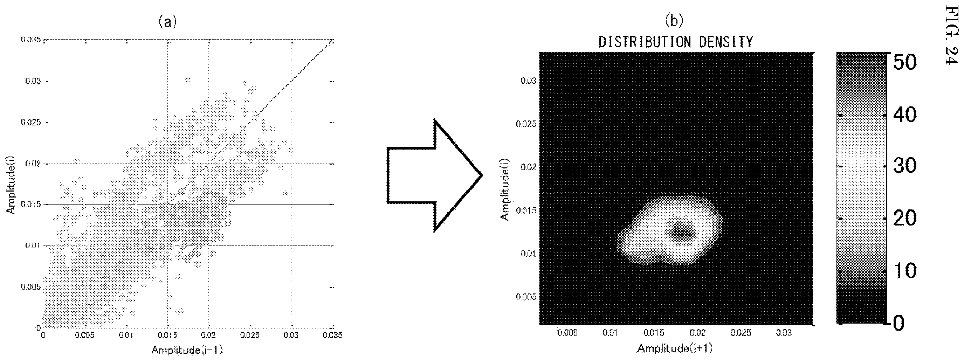

An analysis target of the physiological phenomenon estimation means 250 of this embodiment is the waveform RC1 which is the first pseudo heart sound waveform. An analyzing method, which is the same as that of the aforesaid blood pressure fluctuation estimation means 240, is to plot amplitudes (i, i+1) of two adjacent waveform components out of waveform components corresponding to one cardiac cycle of the waveform RC1 which is the first pseudo heart sound waveform, in a time-series manner in a coordinate system with one of the amplitudes taken on the axis of abscissas and the other taken on the axis of ordinates, and estimate a desire to void from a variance state of a group of plotted points (refer to FIG. 23 and FIGs. 24).

A desire to void correlates with blood pressure fluctuation, and can also be estimated from the second pseudo heart sound waveform in which the pseudo heart sound is made clearer, used in the above-described embodiment, but for quick processing, the analysis target in this embodiment is the waveform RC1 which is the first pseudo heart sound waveform. Further, as illustrated in FIG. 23, as the two amplitudes (i, i+1), this embodiment selected amplitudes (A1, A2) of two waveform components sandwiching a bottom B2 located immediately before the lowest bottom (place having the lowest amplitude value in the waveform components in one cardiac cycle) B1, out of waveform components included in one cardiac cycle. The amplitudes of waveform components before and after the highest peak, out of the waveform components included in one cardiac cycle, may be used as in the above-described embodiment. In either case, it is preferable to select and use two waveform components whose amplitudes are large in the amplification phase, according to a predetermined criterion.

The physiological phenomenon estimation means 250 uses amplitudes (A1(i), A2(i+1)) of two waveform components in an amplification phase illustrated in FIG. 23 and plots their amplitude ratio in a coordinate system. FIG. 24(a) illustrates an example thereof, and in the drawing, the dark-colored point group corresponds to this. The other pale-colored point groups are the results of plotting amplitude ratios in an amplification phase before the amplitudes (A1(i), A2(i+1)) of the specific waveform components and in a damping period after the amplitudes (A1(i), A2(i+1)), and since the amplitude ratio of the waveform components in the amplification phase close to the highest peak exhibits the correlation with the blood pressure fluctuation as in the above-described embodiment, a variance state of the pale-colored point group is not used for the estimation of the desire to void. Here, in FIG. 24(b), the plot of the ratio of the amplitudes (A1(i), A2(i+1)) of the waveform components used for the estimation of the desire to void is made clear. This chart results from calculating the distribution density of the plot and representing the number of plots as a contour map of the distribution density.

### - Experimental Example 2

### (Experiment Method)

Healthy subjects in their twenties to thirties (eight males (since the experiment was conducted twice for three subjects among them, the number of obtained experimental data is eleven)) were requested to take water, and a relation between a sensation level of a desire to void and the aforesaid group of the plotted points of the amplitude ratio found by the physiological phenomenon estimation means 250 was studied. The sensation level of the desire to void was classified into "normal time" which is a state where the subject felt no desire to void after taking water, "post-perception time" which is an instant at which a desire to void was thereafter felt (that is, an instant at which the subject became conscious of a first desire to void (FDV)), and "pre-perception time" which is an instant immediately before the subject was conscious of the first desire to void. Further, an instant at which the subject was conscious of the maximum desire to void (MDV) which is the limit of suppressing the desire to void was classified as "limit time", and an instant at which the subject was conscious of a strong desire to void (SDV) between the "post-perception time" and the "limit time" was classified as "suppression time".

In the experiment, the subjects were each seated in an automobile seat for experiment in which the biosignal measurement device 1 used in trade name "Sleep Buster" manufactured by Delta Tooling Co., Ltd. was attached to its seat back part, and back sound and vibration information was collected while the subjects were in a resting state and a sitting posture. Data of the back sound and vibration information was analyzed by the biological state estimation device 100 which is a computer. At the same time, an electrocardiogram (hereinafter, "ECC", a measuring instrument: Bedside Monitor BSM-2300 series Life Scope I manufactured by Nihon Kohden Corporation) was measured, and a systolic blood pressure (highest blood pressure) and a diastolic blood pressure (lowest blood pressure) of the upper arm were measured every fifteen minutes, using an upper arm blood pressure monitor for home use (OMRON HEM-7051).

After urination, the subjects were each seated in the aforesaid automobile seat for experiment and the experiment was started, and after 45 minutes passed from the start of the experiment, they took 500 ml water in fifteen minutes, were kept in the resting state until declaring the maximum desire to void (limit time), and urinated after declaring the maximum desire to void, a urinary amount was measured, and then the experiment was finished.

### (Experimental Result)

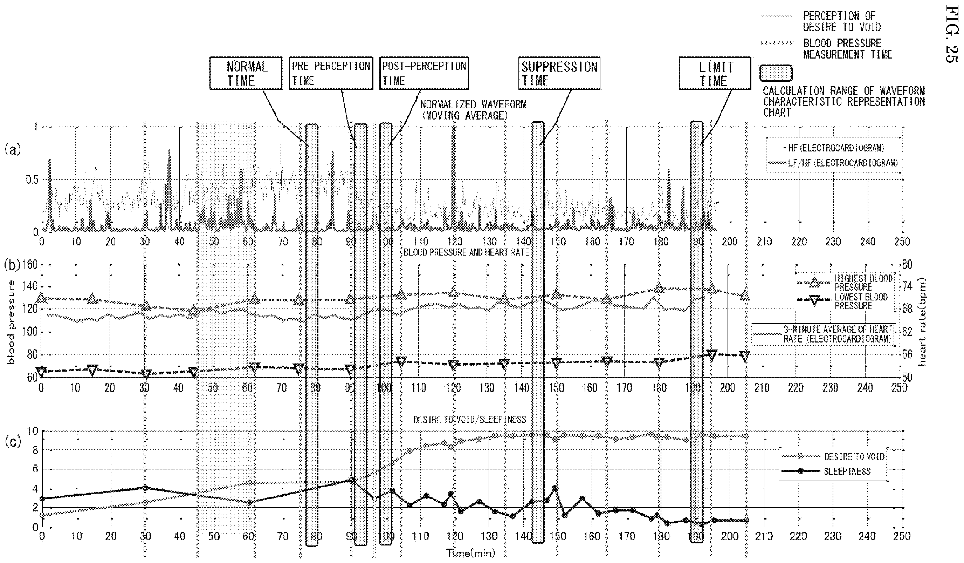

FIGs. 25 are charts illustrating analysis results of the subject A who had a relatively small influence of sleepiness. FIG. 25(a) illustrates graphs of time-series waveforms of HF and LF/HF of the electrocardiogram, FIG. 25(b) illustrates graphs of time-series variations of the highest blood pressure and the lowest blood pressure, and a variation in heart rate found every fifteen minutes, and FIG. 25(c) illustrates graphs of the self-declared levels of the desire to void and sleepiness. From these graphs, it is seen that, at the "limit time", in a period where LF/HF is relatively stable, HF rises and the highest blood pressure and the lowest blood pressure both rise. Further, it is seen that the highest blood pressure and the lowest blood pressure both tend to be higher at the "post-perception time", the "suppression time", and the "limit time" than at the "normal time" and the "pre-perception time".

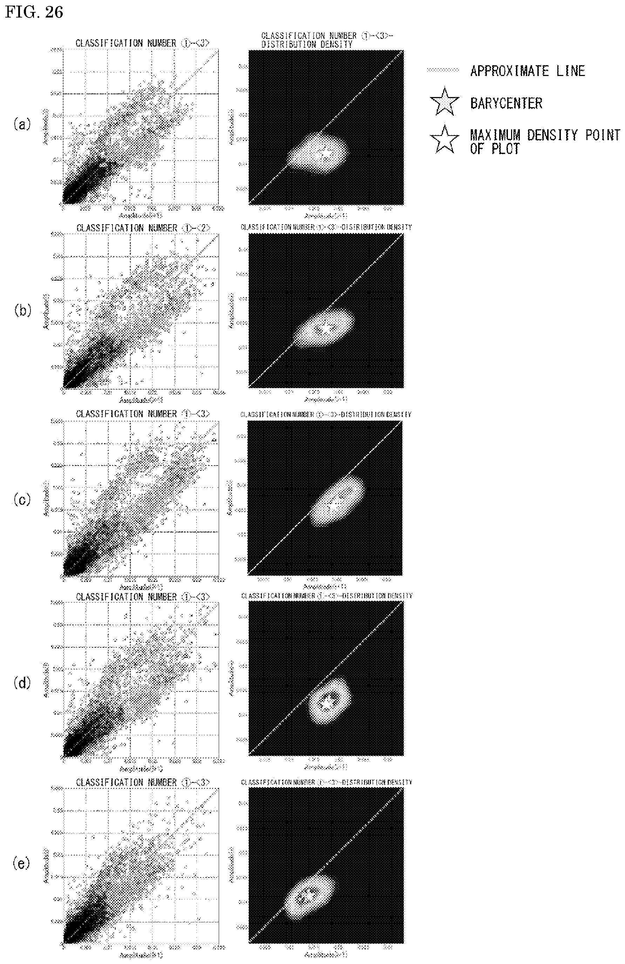

FIGs. 26 illustrate output results found by the physiological phenomenon estimation means 250 of this embodiment, (a) illustrating the output result at the "normal time", (b) illustrating the output result at the "pre-perception time", (c) illustrating the output result at the "post-perception time", (d) illustrating the output result at the "suppression time", and (e) illustrating the output result at the "limit time". Further, in FIGs. 26(a) to (e), the left drawings each illustrate a plot chart of the amplitude ratio and the right drawings each illustrate a distribution density contour map of the point group of the ratio of the predetermined amplitudes (A1(i), A2(i+1)), created from the left plot chart.

From FIGs. 26, it is seen that, at the "suppression time" and the "limit time", the width of the point group of the ratio of the amplitudes (A1(i), A2(i+1)) tends to be smaller than that before these times, and the distribution tends to concentrate at one point. Further, as the desire to void is felt stronger, the center of this point group tends to be more in the direction of the origin of coordinates. Therefore, by setting thresholds of the area of the point group, the position of the center point, and the like on the coordinates, the physiological state estimation means 250 is capable of estimating the level of the desire to void.

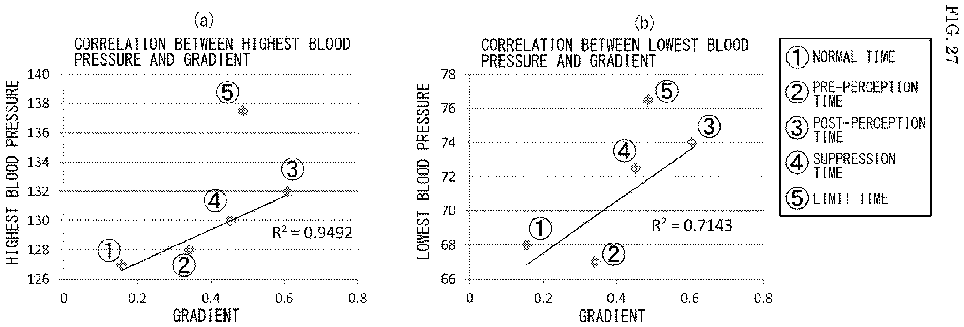

FIGs. 27 are charts illustrating the correlations of a gradient of an approximate line of the point group of the ratio of the amplitudes (A1(i), A2(i+1)) found in FIGs. 26 with the highest blood pressure (FIG. 27(a)) and with the lowest blood pressure (FIG. 27(b)). Note that, as data of the blood pressure, a blood pressure when 75 minutes passed was adopted for the "normal time", a blood pressure when 90 minutes passed was adopted for the "pre-perception time", a blood pressure when 105 minutes passed was adopted for the "post-perception time", an average value of blood pressures when 135 minutes passed and when 195 minutes passed was adopted for the "suppression time", and a blood pressure when 180 minutes passed and when 195 minutes passed was adopted for the "limit time". From FIGs. 27, it is seen that the highest blood pressure and the lowest blood pressure both deviate from the approximate lines in FIGs. 27 only at the "limit time". Therefore, when capable of determining the appearance of a singular point greatly deviating from the correlation between the gradient of the approximate line of the point group of the ratio of the amplitudes (A1(i), A2(i+1)) found in FIGs. 26 and the blood pressure, the physiological state estimation means 250 is capable of estimating that the subject has a desire to void on the "limit time" level.

In this experimental example, the blood pressure monitor was used to measure the blood pressure, but it is also possible to estimate the desire to void by the method of the physiological phenomenon estimation means 250 of this embodiment which finds the singular point as illustrated in FIGs. 27 by using the output result of the blood pressure fluctuation estimated by the blood pressure fluctuation estimation means 240 of the above-described embodiment.

Next, a non-claimed embodiment where a means different from that of the embodiment described using FIG. 22 to FIGs. 27 is adopted as the physiological phenomenon estimation means 250 will be described. The physiological phenomenon estimation means 250 of this embodiment adopts a means which estimates a desire to void by comparing patterns of amplitude changes in cardiac cycles of a pseudo heart sound waveform, instead of using the amplitude ratio of the two waveform components. Note that a pseudo heart sound waveform to be analyzed in this embodiment is also the aforesaid RC1 waveform which is the first pseudo heart sound waveform.

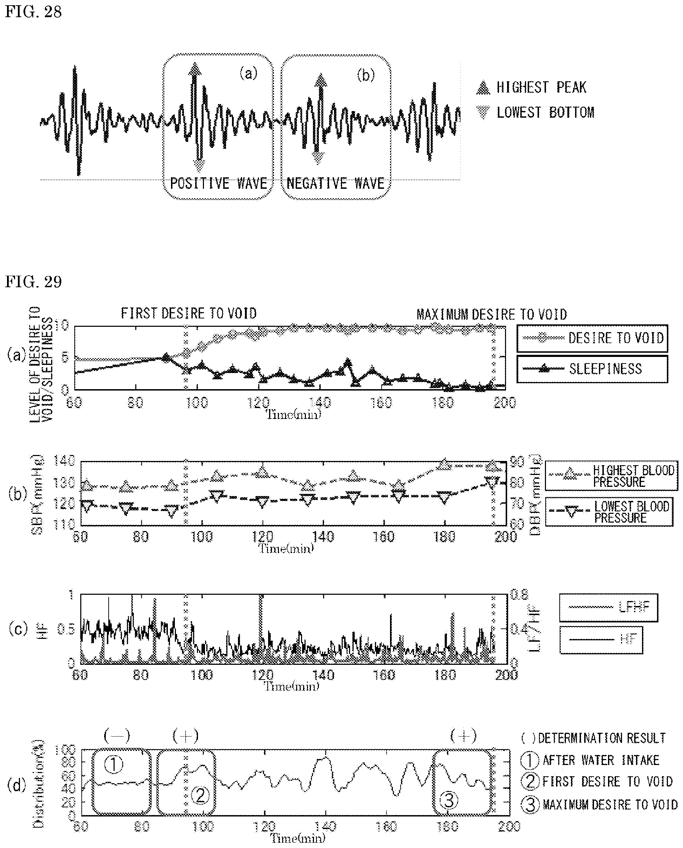

As illustrated in FIG. 28, when focusing on cardiac cycles (two surrounded regions (a), (b) in FIG. 28) of the first pseudo heart sound waveform, in waveform components of the pseudo first sound corresponding to the first heart sound, there exist: a waveform pattern in the region (a) presenting a change in which a point with the lowest amplitude (lowest bottom) appears immediately after the appearance of a point with the highest amplitude (highest peak) (hereinafter, referred to as a "positive waveform pattern"); and a waveform pattern in the region (b) presenting a change in which a point with the lowest amplitude (lowest bottom) appears immediately before the appearance of a point with the highest amplitude (highest peak) (hereinafter, referred to as a "negative waveform pattern"). In this embodiment, these two kinds of waveform patterns were used to estimate a desire to void.

That is, the physiological phenomenon estimation means 250 of this embodiment is set to estimate the desire to void from an appearance ratio of the positive waveform pattern and the negative waveform pattern during a predetermined period. This will be described using data of the subject A in the above embodiment.

FIGs. 29 illustrate time-series variations of subjective evaluations of a desire to void and sleepiness of the subject A (FIG. 29(a)), the highest blood pressure (SBP) and the lowest blood pressure (DBP) (FIG. 29(b)), and HF and LF/HF (FIG. 29(c)), in a period from 60 minutes up to 200 minutes after the start of the experiment, and FIG. 29(d) illustrates a time-series variation of the appearance ratio of the negative waveform pattern. The time-series variation of the appearance ratio of the negative waveform pattern was found by calculating the appearance ratio of the negative waveform pattern in a 180-second period every thirty seconds and plotting the results.

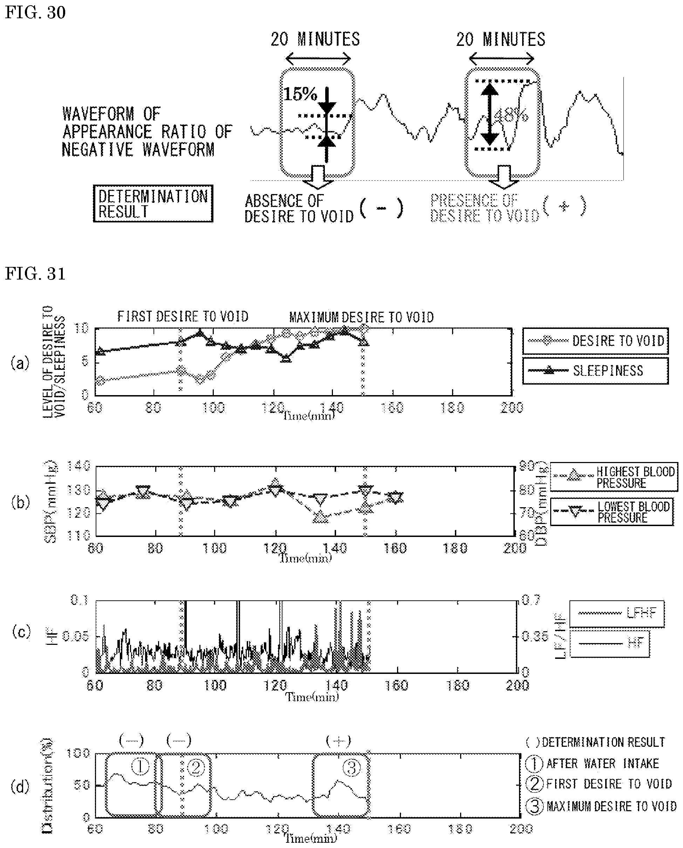

As illustrated in FIG. 29(d), the appearance ratio of the negative waveform pattern fluctuates only a little from the start of the experiment up to the onset of the first desire to void, but the appearance ratio greatly fluctuates after the onset of the first desire to void. The timing of this change agrees with the fact that HF in FIG. 29(c) presents a noticeable decreasing tendency from the onset of the first desire to void. This is because the parasympathetic nerves are involved in the contraction of the bladder and the relaxation of the sphincter, and by using the appearance ratio of the negative waveform pattern which noticeably fluctuates from the onset of the desire to void similarly to HF, it is possible to detect a bodily change ascribable to the desire to void. Therefore, in this embodiment, the physiological phenomenon estimation means 250 was set to find the appearance ratio of the negative waveform pattern and determine "presence of desire to void" when the appearance ratio agreed with a preset determination criterion. Examples of the determination criterion include an increase rate of the appearance ratio of the negative waveform pattern in a predetermined time range. In this embodiment, as illustrated in FIG. 30, it was set that the determination of "presence of desire to void" was made when a difference between the minimum value and the maximum value of the appearance ratio of the negative waveform pattern increased by 30% or more during twenty minutes immediately before the determination time.

Table 7 shows the correlation between the determination of the presence/absence of a desire to void according to the determination criterion illustrated in FIG. 30 regarding the data of the subjects in the above-described experimental example 2 and the subjective evaluation of the subjects. Specifically, "absence of desire to void" in the subjective evaluation is a state after the intake of 500 ml water, and "presence of desire to void" is a state at the onset of the first desire to void (when a weak desire to void is felt) and at the time of the maximum desire to void (when a strong desire to void is felt), and based on the timings of these, the determination adopting the determination criterion in FIG. 30 by the physiological phenomenon estimation means 250 was performed, and the both were compared.

**[Table 7]**

| | | Determination result by physiological phenomenon estimation means 250 | |
|---|---|---|---|
| | | Presence of desire to void (+)) | Absence of desire to void (-) |
| State of subj ect (subjective) | Absence of desire to void (after intake of water) | 18% (number determined 2) | 82% (number determined 9) |
| | Presence of desire to void (first desire to void and maximum desire to void)) | 68% (number determined 15) | 32% (number determined 7) |

Percentage of correct answers was 82% for "presence of desire to void" and 68% for "absence of desire to void". The result of the Fisher's exact test was p = 0.01, which is in the range of p < 0.05, and therefore, a significant correlation was recognized between the presence/absence of the subjective desire to void of the subjects and the determination of the presence/absence of the desire to void based on the appearance ratio of the negative waveform pattern.

However, determination accuracy differs to a certain degree depending on each subject, and among the subjects, the subject A whose experimental results are illustrated in FIGs. 29 had a high percentage of correct answers. FIGs. 31 illustrate the experimental results of the subject B whose percentage of correct answers was relatively low. When the both are compared, as for the subject A, influences by the desire to void, such as a decrease in HF and a blood pressure rise, appear in the biological indexes from the onset of the first desire to void as illustrated in FIGs. 29, and it is thought that the noticeable fluctuation of the appearance ratio of the negative waveform pattern also represents a bodily state change accompanying the desire to void. On the other hand, in the case of the subject B, as illustrated in FIG. 31(b), the blood pressure repeatedly fluctuates to drop as the sleepiness becomes stronger and rise as the sleepiness becomes weaker up to an instant when 135 minutes pass, but at the time of the maximum desire to void, the blood pressure rises irrespective of the strong sleepiness. Further, as illustrated in FIG. 31(c), HF and LF/HF do not change much from the start of the experiment, but from an instant before the declaration of the maximum desire to void, HF presents a decreasing tendency and LF/HF presents an increasing tendency. From these, it can be said that the biological indexes of the subject B reflect the influence by the desire to void at the time of the maximum desire to void, but because the sleepiness having a stronger influence on the bodily state change than the desire to void is dominant, the appearance ratio of the negative waveform pattern does not greatly fluctuate at the time of the first desire to void, which is a possible reason for the inability to detect the desire to void.

It is thought that the appearance ratio of the negative waveform pattern found from the pseudo heart sound waveform in this embodiment varies by being influenced by a reflected wave of a pulse wave generated from the heart, that is, it varies when arterial walls are hardened due to an influence of a blood pressure rise or the like and accordingly a propagation velocity of the reflected wave becomes faster. Therefore, the early return of the reflected wave due to the blood pressure rise accompanying the onset of the desire to void appears as a great fluctuation of the appearance ratio of the negative waveform pattern, making it possible to detect the desire to void.

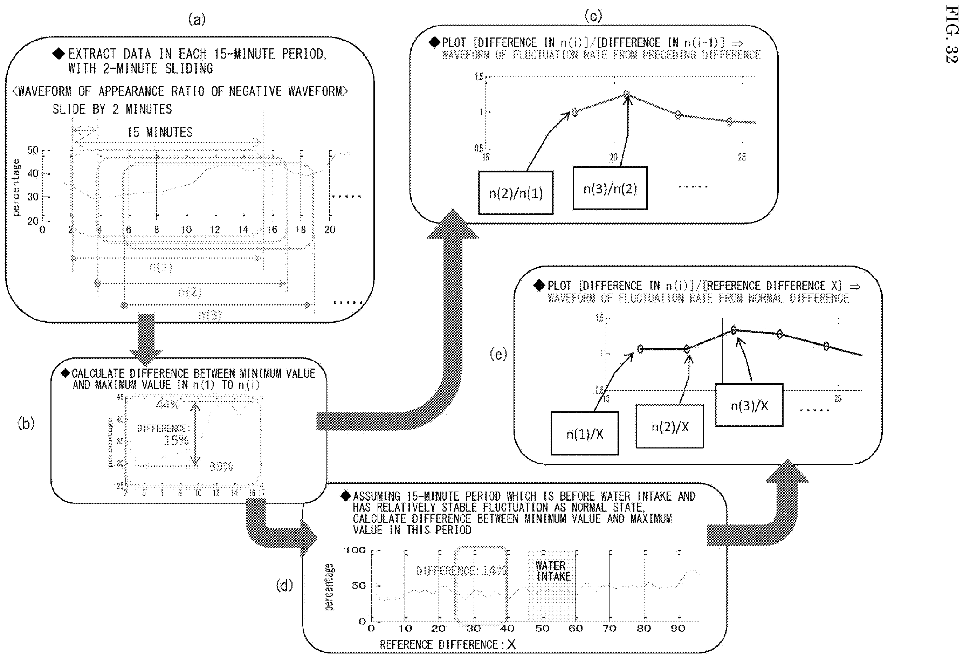

Next, a method to more quickly determine an instant when the desire to void occurs, by using the method to determine the desire to void from the appearance ratio of the negative waveform pattern described in FIG. 28 to FIGs. 31 will be described based on FIGs. 32.

First, as illustrated in FIG. 32(a), a difference between the maximum value and the minimum value of the appearance ratio of the negative waveform pattern is found in a predetermined time range as in the above. Note that, in the above experimental example, the predetermined time range is set to twenty minutes, but in the calculation example in FIGs. 32, it is set to fifteen minutes, and while the predetermined time range is slid by two minutes each time, the difference is sequentially found using data in the respective predetermined time ranges (n(1) to n(i)). For example, as illustrated in FIG. 32(b), the difference is calculated as 15% in a fifteen-minute period from a two-minute instant up to a seventeen-minute instant, and such calculation is conducted sequentially. Next, as illustrated in FIG. 32(c), an output difference in data of a give fifteen-minute period (n(i)) and an output difference in data of an immediately preceding fifteen-minute period (n(i-1)) are compared, and the result is plotted as a fluctuation rate from preceding difference. Further, from data in a given fifteen-minute period during the normal time before the desire to void is felt, a difference serving as a reference (reference difference) X is found (FIG. 32(d)), and as illustrated in FIG. 32(e), a ratio in the given fifteen-minute period (n(i)) to the reference difference (X) is calculated, and the result is plotted as a fluctuation rate from normal difference. Then, a waveform of the fluctuation rate from preceding difference in FIG. 32(c) and a waveform of the fluctuation rate from normal difference in FIG. 32(e) are compared.

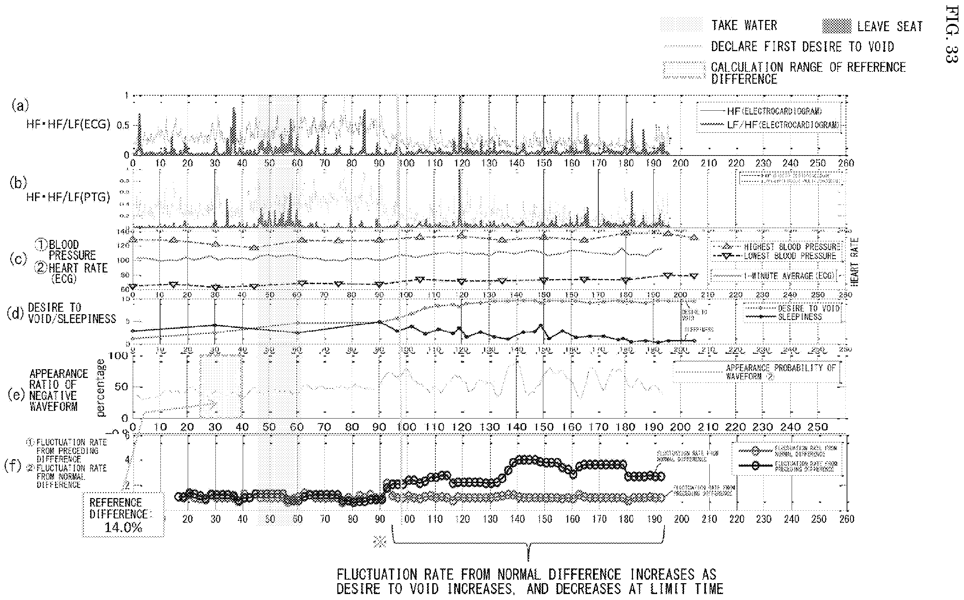

FIGs. 33 illustrate examples where the method in FIGs. 32 is applied to the aforesaid experimental data. Note that, as the data during the normal time, data in a fifteen-minute period which was before the water intake and had a relatively stable waveform of the appearance ratio of the negative waveform was selected for use, and in this example, the reference difference X was set to 14%. The graphs illustrated in FIG. 33(f) are the results. From these graphs, it is seen that the fluctuation rate from normal difference increases as the desire to void increases. Therefore, the use of the graphs in FIG. 33(f) makes it possible to quickly determine the occurrence of the desire to void at an instant when the fluctuation rate from normal difference increases, while sequentially finding the appearance ratio of the negative waveform pattern.

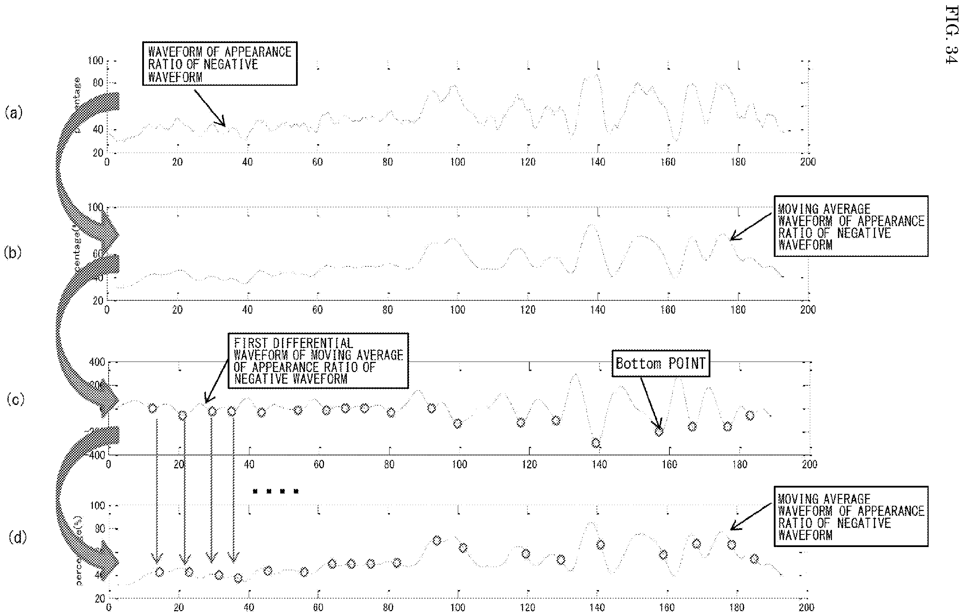

Next, another analysis method to detect a change in the biological state using the appearance ratio of the negative waveform pattern will be described based on FIGs. 34.

First, in the same manner as in the above, the time-series waveform of the appearance ratio of the negative waveform is found (FIG. 34(a)). Thereafter, moving average calculation is performed with a time window being set to, for example, 240 seconds, to find a moving average waveform of the appearance ratio of the negative waveform (FIG. 34(b)). In order to find starting points of periods of the moving average waveform of the appearance ratio of the negative waveform, the moving average waveform is differentiated, and bottom points of its amplitude are extracted (FIG. 34(c)). Next, on the moving average waveform of the appearance ratio of the negative waveform in FIG. 34(b), the bottom points extracted in FIG. 34(c) are plotted (FIG. 34(d)), and fluctuations of the amplitude, period, and baseline of the moving average waveform of the appearance ratio of the negative waveform are found using the data in FIG. 34(d).

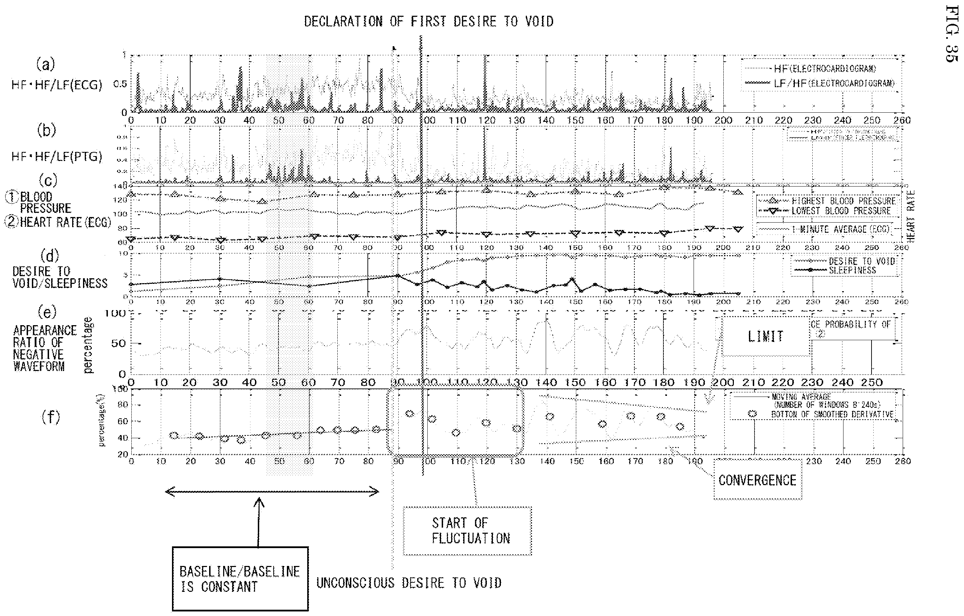

FIGs. 35 are experimental data of the same subject as that in FIGs. 33, FIG. 35(f) illustrating a graph in which bottom points each being the starting point of the period are plotted on the moving average waveform of the appearance ratio of the negative waveform. Up to an instant of the declaration of the first desire to void, the baseline is substantially constant and, in addition, the frequency is stable and the amplitude is small. On the other hand, from the instant of the declaration of the first desire to void, fluctuations occur, with the amplitude becoming larger, and the period becoming longer. On the other hand, after 140 minutes pass, as the time of the maximum desire to void (limit time) is approached, a convergence tendency of the amplitude is seen.

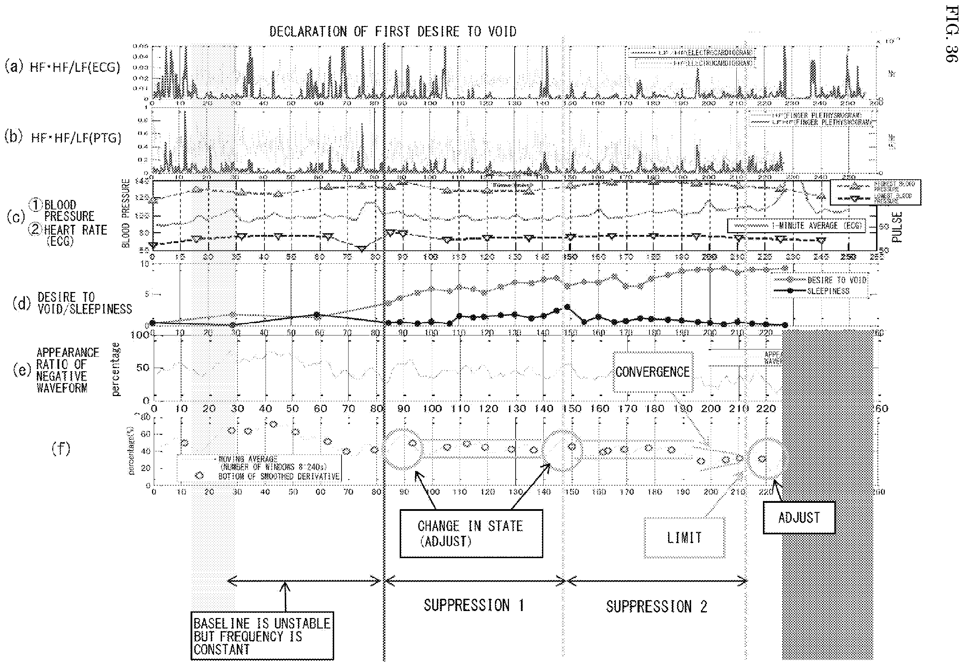

FIGs. 36 are different experimental data of the same subject as that in FIGs. 35. From the lowest graph in FIG. 36(f), it is seen that, up to the instant of the declaration of the first desire to void, the frequency is similarly substantially constant even though the baseline is unstable. On the other hand, at the instant of the declaration of the first desire to void, the amplitude greatly changes and the period fluctuates. Thereafter, the period becomes stable but the amplitude increases again when about 140 to 150 minutes pass. At this instant, as is obvious from the graphs of the desire to void and sleepiness (FIG. 36(d)), the sleepiness fluctuates and the desire to void temporarily decreases. Further, around the time of the maximum desire to void (limit time), a tendency that the amplitude converges and a tendency that it thereafter increases appear. That is, at an instant when the desire to void starts to be felt, an instant when the sleepiness changes, and at the time of the maximum desire to void, the occurrence of some change in the biological state is detected.

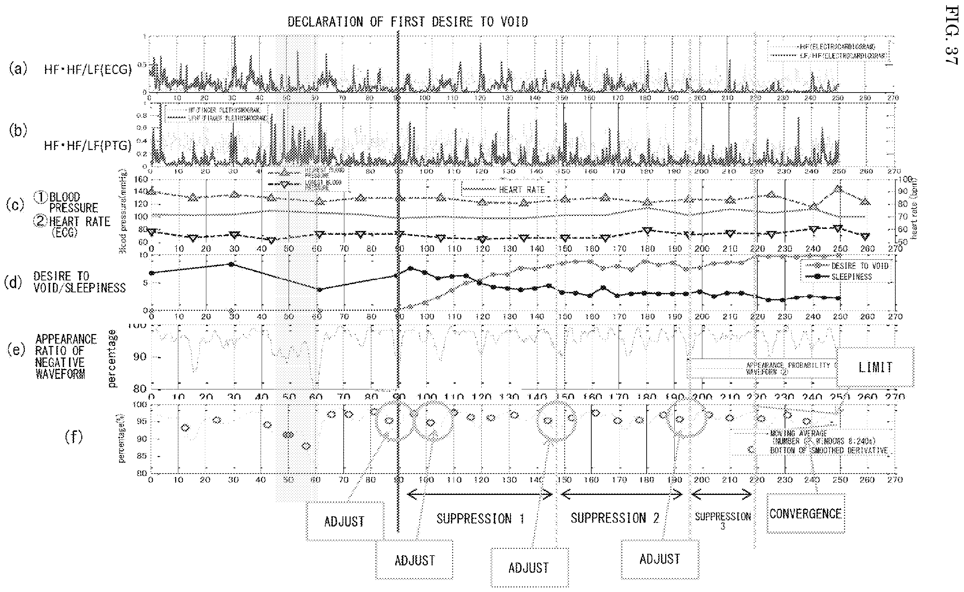

FIGs. 37 are data of a different subject, and as illustrated in FIG. 37(f), the amplitude and the period fluctuate at an instant when the first desire to void is declared, and when about 150 minutes or 190 minutes pass, which is the timing when a certain time has passed while the desire to void is suppressed, the amplitude and the period fluctuate. Then, as the time of the maximum desire to void (limit time) is approached, they present a convergence tendency.

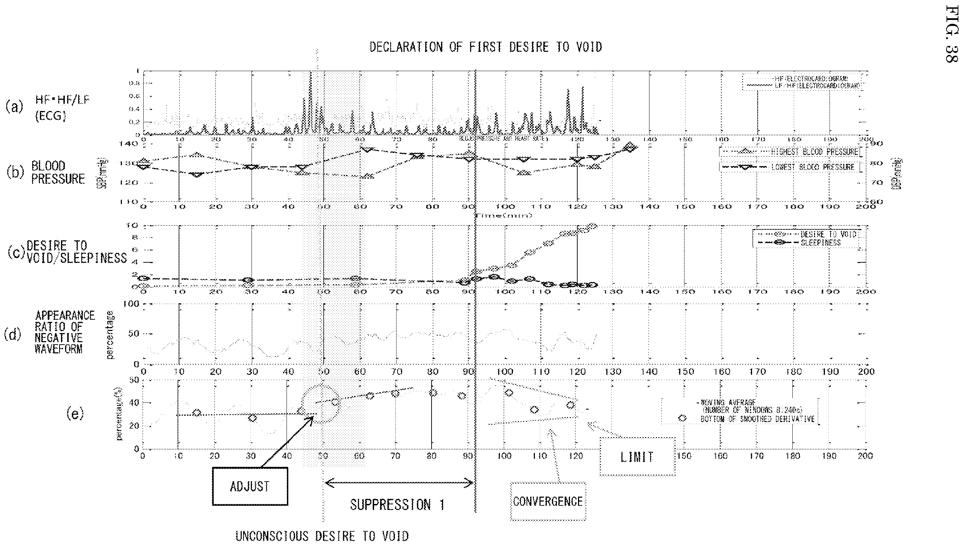

FIGs. 38 are data of still another subject, and from FIG. 38(e), changes in the amplitude and the period and a change in the gradient of the baseline are seen around an instant when fifteen minutes have passed. At this instant, a desire to void is not felt, but these changes correspond to the fact that LF/HF becomes dominant and sympathetic nerve activity becomes active. Then, when the first desire to void is declared around an instant when 90 minutes have passed, the amplitude and the period also fluctuate, and the amplitude presents a convergence tendency near the limit time.

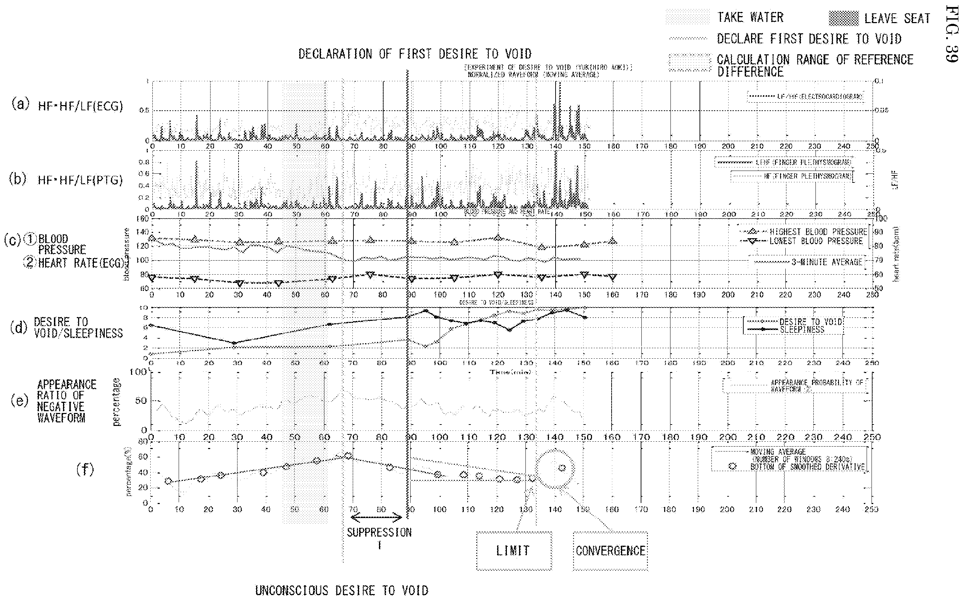

FIGs. 39 are data of yet another subject. As illustrated in FIG. 39(d), sleepiness of this subject increases from an instant thirty minutes after the start of the experiment up to around an instant when the subject declares the first desire to void. From FIG. 39(f), it is seen that up to an instant when about seventy minutes have passed, the gradient of the baseline rises as the sleepiness increases. Thereafter, as the vicinity of the instant of the declaration of the first desire to void is approached, the gradient of the baseline changes and the fluctuation of the period is seen, and further the amplitude presents a convergence tendency near the limit time.

Variations of the fluctuations of the amplitude, the period, the baseline, and so on of the moving average waveform of the appearance ratio of the negative waveform appear in accordance with the change in the desire to void as described above, which indicates that from these fluctuations, the desire to void can be detected, but in addition, their fluctuations are seen according to a change in the sleepiness and autonomic nervous activity, and these fluctuations are indexes usable for detecting various changes in the biological states.

### Explanation of Reference Signs

- 1: biological signal measurement device
- 11: core pad
- 12: spacer pad
- 13: sensor
- 100: biological state estimation device
- 200: biological state estimation means
- 210: back sound and vibration information processing means
- 220: pseudo heart sound waveform calculation means
- 230: low-frequency time-series waveform calculation means
- 240: blood pressure fluctuation estimation means
- 250: physiological state estimation means

## Claims

1. A biological state estimation device (100) configured to estimate a biological state of a person by using a biosignal, the biological state estimation device (100) comprising
a biological state estimation means (200) configured to estimate the biological state by using a pseudo heart sound waveform corresponding to a period of heart sound, and obtaining the pseudo heart sound waveform by processing biological sound and vibration information which is collected as the biosignal from a back of the person and fluctuates according to a flow rate of blood pumped out from the heart, **characterized in that**
the biological state estimation means (200) is furthermore configured to plot amplitudes (i, i+1) of adjacent two waveform components in one cardiac cycle of the pseudo heart sound waveform in a time-series manner in a coordinate system, with one of the amplitudes taken on the axis of abscissas and the other taken on the axis of ordinates, and to estimate the biological state from a variance state of a group of the plotted points, and the biological state estimation means (200) is configured to estimate the biological state from a gradient of an approximate line of the group of the plotted points by comparing the gradient angle of the approximate line of the group of the plotted points with a correlation data of a blood pressure and the gradient angle of the approximate line which is stored in a storage in advance, wherein
the biological state estimation includes a blood pressure fluctuation estimation which estimates a state of blood pressure fluctuation as the biological state, and wherein
the pseudo heart sound waveform is a second pseudo heart sound waveform, Pseudo-PCG, including pseudo first and second sounds,
the second pseudo heart sound waveform is obtained by:
filtering (S11) an original waveform, RC0, of the back sound and vibration information by a band pass filter with a center frequency of around 20 Hz to obtain a first pseudo heart sound waveform, RC1;
giving a distortion to the first pseudo heart sound waveform, RC1, by a clipping processing (S12), wherein a threshold of the amplitude at the time of the clipping processing is set at such a position that a time width corresponding to that between first and second heart sounds in a heart sound waveform can be secured, to obtain a clipped waveform, Clip;
and applying a high pass filter (S13) to the clipped waveform, Clip, to exclude signals except those at clipped parts and emphasize the pseudo first and second sounds.

2. A computer program comprising instructions which, when the program is executed by a computer, cause the computer to carry out a biological state estimation procedure which estimates the biological state by using a pseudo heart sound waveform corresponding to a period of heart sound, comprising the steps of:
obtaining the pseudo heart sound waveform by processing biological sound and vibration information which is collected as the biosignal from a back of the person and fluctuates according to a flow rate of blood pumped out from the heart, and being characterized that the steps of
plotting amplitudes (i, i+1) of adjacent two waveform components in one cardiac cycle of the pseudo heart sound waveform in a time-series manner in a coordinate system, with one of the amplitudes taken on the axis of abscissas and the other taken on the axis of ordinates,
estimating the biological state from a variance state of a group of the plotted points, and
estimating the biological state from a gradient of an approximate line of the group of the plotted points by comparing the gradient angle of the approximate line of the group of the plotted points with a correlation data of a blood pressure and the gradient angle of the approximate line which is stored in a storage in advance, wherein
the biological state estimation procedure includes a blood pressure fluctuation estimation procedure which estimates a state of blood pressure fluctuation as the biological state, and
the pseudo heart sound waveform is a second pseudo heart sound waveform, Pseudo-PCG, including pseudo first and second sounds,
the second pseudo heart sound waveform is obtained by:
filtering (S11) an original waveform, RC0, of the back sound and vibration information by a band pass filter with a center frequency of around 20 Hz to obtain a first pseudo heart sound waveform, RC1;
giving a distortion to the first pseudo heart sound waveform, RC1, by a clipping processing, wherein a threshold of the amplitude at the time of the clipping processing is set at such a position that a time width corresponding to that between first and second heart sounds in a heart sound waveform can be secured, to obtain a clipped waveform, Clip;
and applying a high pass filter (S13) to the clipped waveform, Clip, to exclude signals except those at clipped parts and emphasize the pseudo first and second sounds.

## Patentansprüche

1. Vorrichtung (100) zur Schätzung eines biologischen Zustands, die dazu konfiguriert ist, unter Verwendung eines Biosignals einen biologischen Zustand einer Person abzuschätzen, wobei die Vorrichtung (100) zur Schätzung eines biologischen Zustands
ein Mittel (200) zur Schätzung eines biologischen Zustands umfasst, das dazu konfiguriert ist, den biologischen Zustand unter Verwendung einer Pseudo-Herztonwellenform, die einer Herztonsperiode entspricht, abzuschätzen, und durch Erhalten der Pseudo-Herztonwellenform durch Verarbeiten biologischer Schall- und Vibrationsinformationen, die als das Biosignal von einem Rücken der Person gesammelt werden und entsprechend einer Strömungsgeschwindigkeit von aus dem Herzen gepumptem Blut schwanken, **dadurch gekennzeichnet, dass**
das Mittel (200) zur Schätzung eines biologischen Zustands außerdem dazu konfiguriert ist, Amplituden (i, i+1) benachbarter zweier Wellenformkomponenten in einem Herzzyklus der Pseudo-Herztonwellenform in einer Zeitreihenart in einem Koordinatensystem aufzutragen,
wobei eine der Amplituden auf der Abszissenachse aufgetragen wird und die andere auf der Ordinatenachse aufgetragen wird, und um den biologischen Zustand aus einem Varianzzustand einer Gruppe der aufgetragenen Punkte abzuschätzen, und das Mittel (200) zur Schätzung eines biologischen Zustands dazu konfiguriert ist, den biologischen Zustand aus einer Steigung einer Näherungsgeraden der Gruppe der aufgetragenen Punkte abzuschätzen, indem es den Steigungswinkel der Näherungsgeraden der Gruppe der aufgetragenen Punkte mit Korrelationsdaten eines Blutdrucks und den Steigungswinkels der Näherungsgeraden, die vorab in einem Speicher gespeichert wird, vergleicht, wobei
die Schätzung eines biologischen Zustands eine Schätzung einer Blutdruckschwankung einschließt, die einen Zustand einer Blutdruckschwankung als den biologischen Zustand schätzt, und wobei
die Pseudo-Herztonwellenform eine zweite Pseudo-Herztonwellenform, Pseudo-PCG, ist, einschließlich erster und zweiter Pseudotöne,
wobei die zweite Pseudo-Herztonwellenform erhalten wird durch:
Filtern (S11) einer ursprünglichen Wellenform, RC0, der Hintergrundschall- und VibrationsInformationen durch einen Bandpassfilter mit einer Mittenfrequenz von etwa 20 Hz, um eine erste Pseudo-Herztonwellenform, RC1, zu erhalten;
Verzerren der ersten Pseudo-Herztonwellenform, RC1, durch eine Beschneidungsverarbeitung (S12), wobei
ein Schwellenwert der Amplitude zum Zeitpunkt der Beschneidungsverarbeitung auf eine solche Position eingestellt wird, dass eine Zeitbreite, die derjenigen zwischen ersten und zweiten Herztönen in einer Herztonwellenform entspricht, sichergestellt werden kann, um eine beschnittene Wellenform, Clip, zu erhalten; und
Anwenden eines Hochpassfilters (S13) auf die beschnittene Wellenform, Clip, um Signale außer denen an beschnittenen Teilen auszuschließen und die ersten und zweiten Pseudotöne hervorzuheben.

2. Computerprogramm, das Anweisungen umfasst, die, wenn das Programm von einem Computer ausgeführt wird, den Computer veranlassen, ein Verfahren zur Schätzung eines biologischen Zustands auszuführen, das den biologischen Zustand unter Verwendung einer Pseudo-Herztonwellenform abschätzt, die einer Herztonperiode entspricht, umfassend die Schritte des:
Erhaltens der Pseudo-Herztonwellenform durch Verarbeiten biologischer Schall- und Vibrationsinformationen, die als das Biosignal von einem Rücken der Person gesammelt werden und entsprechend einer Strömungsgeschwindigkeit von aus dem Herzen gepumptem Blut schwanken, und **gekennzeichnet durch** die Schritte des
Auftragens von Amplituden (i, i+1) benachbarter zweier Wellenformkomponenten in einem Herzzyklus der Pseudoherztonwellenform in einer Zeitreihenart in einem Koordinatensystem, wobei eine der Amplituden auf der Abszissenachse aufgetragen wird und die andere auf der Ordinatenachse aufgetragen wird,
Schätzens des biologischen Zustands aus einem Varianzzustand einer Gruppe der aufgetragenen Punkte, und
Schätzens des biologischen Zustands aus einem Gradienten einer Näherungsgeraden der Gruppe der aufgetragenen Punkte **durch** Vergleichen des Steigungswinkels der Näherungsgeraden der Gruppe der aufgetragenen Punkte mit Korrelationsdaten eines Blutdrucks und des Steigungswinkels der Näherungsgeraden, der vorab in einem Speicher gespeichert wird, wobei
das Verfahren zur Schätzung eines biologischen Zustands ein Verfahren zur Schätzung einer Blutdruckschwankung einschließt, das einen Zustand einer Blutdruckschwankung als den biologischen Zustand schätzt, und
die Pseudo-Herztonwellenform eine zweite Pseudo-Herztonwellenform, Pseudo-PCG, ist, einschließlich erster und zweiter Pseudotöne,
wobei die zweite Pseudo-Herztonwellenform erhalten wird **durch**:
Filtern (S11) einer ursprünglichen Wellenform, RC0, der Hintergrundschall- und Vibrationsinformationen **durch** einen Bandpassfilter mit einer Mittenfrequenz von etwa 20 Hz, um eine erste Pseudo-Herztonwellenform, RC1, zu erhalten;
Verzerren der ersten Pseudo-Herztonwellenform, RC1, **durch** eine Beschneidungsverarbeitung, wobei
ein Schwellenwert der Amplitude zum Zeitpunkt der Beschneidungsverarbeitung auf eine solche Position eingestellt wird, dass eine Zeitbreite, die derjenigen zwischen ersten und zweiten Herztönen in einer Herztonwellenform entspricht, sichergestellt werden kann, um eine beschnittene Wellenform, Clip, zu erhalten; und
Anwenden eines Hochpassfilters (S13) auf die beschnittene Wellenform, Clip, um Signale außer denen an beschnittenen Teilen auszuschließen und die ersten und zweiten Pseudotöne hervorzuheben.

## Revendications

1. Dispositif d'estimation d'6tat biologique (100) configuré pour estimer un 6tat biologique d'une personne en utilisant un signal biologique, le dispositif d'estimation d'6tat biologique (100) comprenant
un moyen d'estimation d'6tat biologique (200) configuré pour estimer l'état biologique en utilisant une forme d'onde de pseudo-bruits cardiaques correspondant à une période de bruits cardiaques, et obtenir la forme d'onde de pseudo-bruits cardiaques en traitant des informations de bruits et de vibrations biologiques qui sont collectées en tant que le signal biologique à partir d'un dos de la personne et fluctuent en fonction d'un débit de sang pomp6 à partir du coeur, **caractérisé en ce que**
le moyen d'estimation d'6tat biologique (200) est en outre configuré pour tracer des amplitudes (i, i+1) de deux composantes de forme d'onde adjacentes dans un cycle cardiaque de la forme d'onde de pseudo-bruits cardiaques à la manière d'une série temporelle dans un système de coordonnées,
avec une des amplitudes prise sur I'axe des abscisses et I'autre prise sur I'axe des ordonnées, et pour estimer l'état biologique à partir d'un état de variance d'un groupe de points tracés, et le moyen d'estimation d'6tat biologique (200) est configuré pour estimer l'état biologique à partir d'un gradient d'une ligne approchée du groupe des points tracés en comparant I'angle de gradient de la ligne approchée du groupe des points tracés avec des données de corrélation d'une pression art6rielle et I'angle de gradient de la ligne approchée qui est stockée dans un stockage à I'avance, dans lequel
l'estimation d'6tat biologique inclut une estimation de fluctuation de pression art6rielle qui estime un état de fluctuation de pression art6rielle en tant qu'état biologique, et dans lequel
la forme d'onde de pseudo-bruits cardiaques est une seconde forme d'onde de pseudo-bruits cardiaques, Pseudo-PCG, incluant des premiers et seconds pseudo-bruits,
la seconde forme d'onde de pseudo-bruits cardiaques est obtenue comme suit:
filtrer (S11) une forme d'onde originale, RC0, des informations de bruits et de vibrations dorsales par un filtre passe-bande avec une fréquence centrale d'environ 20 Hz pour obtenir une première forme d'onde de pseudo-bruits cardiaques, RC1 ;
donner une distorsion à la première forme d'onde de pseudo-bruits cardiaques, RC1, par un traitement d'écrêtage (S12), dans lequel
un seuil de I'amplitude au moment du traitement d'écrêtage est defini au niveau d'une telle position qu'une largeur temporelle correspondant à celle entre les premiers et seconds bruits cardiaques dans une forme d'onde de bruits cardiaques peut 6tre assurée, pour obtenir une forme d'onde écrêtée, Clip ; et
appliquer un filtre passe-haut (S13) à la forme d'onde écrêtée, Clip, pour exclure des signaux à l'exception de ceux au niveau des parties écrêtées et accentuer les premiers et seconds pseudo-bruits.

2. Programme informatique comprenant des instructions qui, lorsque le programme est exécuté par un ordinateur, amènent I'ordinateur à réaliser une procédure d'estimation d'6tat biologique qui estime l'état biologique en utilisant une forme d'onde de pseudo-bruits cardiaques correspondant à une période de bruits cardiaques, comprenant les étapes suivantes :
obtenir la forme d'onde de pseudo-bruits cardiaques en traitant des informations de bruits et de vibrations biologiques qui sont collectées en tant que le signal biologique à partir d'un dos de la personne et fluctuent en fonction d'un débit de sang pomp6 à partir du coeur et étant **caractérisé par** les étapes de
tracer des amplitudes (i, i+1) de deux composantes de forme d'onde adjacentes dans un cycle cardiaque de la forme d'onde de pseudo-bruits cardiaques à la manière d'une série temporelle dans un système de coordonnées, avec une des amplitudes prise sur I'axe des abscisses et I'autre prise sur I'axe des ordonnées,
estimer l'état biologique à partir d'un état de variance d'un groupe de points tracés, et
estimer l'état biologique à partir d'un gradient d'une ligne approchée du groupe de points tracés en comparant I'angle de gradient de la ligne approchée du groupe de points tracés avec des données de corrélation d'une pression art6rielle et I'angle de gradient de la ligne approchée qui est stockée dans un stockage à I'avance, dans lequel
la procédure d'estimation d'6tat biologique inclut une procédure d'estimation de fluctuation de pression art6rielle qui estime un état de fluctuation de pression art6rielle en tant qu'état biologique, et
la forme d'onde de pseudo-bruits cardiaques est une seconde forme d'onde de pseudo-bruits cardiaques, Pseudo-PCG, incluant des premiers et seconds pseudo-bruits,
la seconde forme d'onde de pseudo-bruits cardiaques est obtenue comme suit:
filtrer (S11) une forme d'onde originale, RC0, des informations de bruits et de vibrations dorsales par un filtre passe-bande avec une fréquence centrale d'environ 20 Hz pour obtenir une première forme d'onde de pseudo-bruits cardiaques, RC1 ;
donner une distorsion à la première forme d'onde de pseudo-bruits cardiaques, RC1, par un traitement d'écrêtage, dans lequel
un seuil de I'amplitude au moment du traitement d'écrêtage est défini au niveau d'une telle position qu'une largeur temporelle correspondant à celle entre les premiers et seconds bruits cardiaques dans une forme d'onde de bruits cardiaques peut 6tre assurée, pour obtenir une forme d'onde écrêtée, Clip ; et
appliquer un filtre passe-haut (S13) à la forme d'onde écrêtée, Clip, pour exclure des signaux à l'exception de ceux au niveau des parties écrêtées et accentuer les premiers et seconds pseudo-bruits.
